# EUROPEAN PATENT APPLICATION

(11) **EP 4 464 699 A1**
(43) Date of publication of application: **20.11.2024**
(21) Application number: 23739925.8
(22) Date of filing: 06.01.2023
(51) Int. Cl.: C07D 401/12, C07D 403/12, A61K 31/506, A61P 35/00

(54) **PYRIMIDINE-2,4-DIAMINE DERIVATIVES AS WELL AS PREPARATION METHOD THEREFOR AND USE THEREOF**

(30) Priority: 14.01.2022 CN 202210044658
(71) Applicant: Lisen Therapeutics, Inc., Wilmington, DE 19803 (US)
(72) Inventor: ZHOU, Ning, Wilmington, DE 19803 (US); CHENG, Tong, Wilmington, DE 19803 (US); XU, Yingjiao, Wilmington, DE 19803 (US); LI, Chengtao, Wilmington, DE 19803 (US); ZOU, Wuxin, Wilmington, DE 19803 (US)
(74) Representative: Lavoix
(86) International application number: PCT/CN2023/071052
(87) International publication number: WO 2023/134582

(57) **Abstract**

Provided are pyrimidine-2,4-diamine derivatives and use thereof. Specifically, the pyrimidine-2,4-diamine derivative are as represented by Formula 1, and a racemate, tautomer, enantiomer, diastereomer, isotope-substituted compound, prodrug or pharmaceutically acceptable salt thereof, a preparation method thereof, and use thereof in the preparation of a medicament.

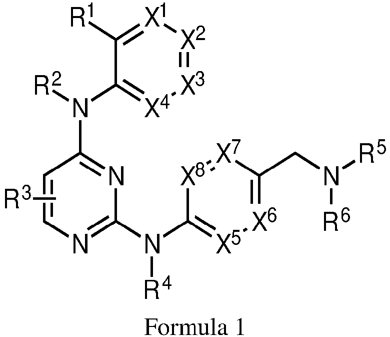

## Description

### CROSS-REFERENCE TO RELATED APPLICATIONS

This application claims priority and benefits of patent application No. 202210044658.7 filed with the State Intellectual Property Office of China on January 14, 2022, the entire content of which is incorporated herein by reference.

### FIELD

The present disclosure belongs to the technical field of treatment of a drug-resistant tumor, and more particularly, relates to a pyrimidine-2,4-diamine derivative, a preparation method therefor, and use thereof.

### BACKGROUND

Cancer has become the second largest disease that seriously threatens human life and health. According to statistics, there are as many as 10 million new cancer patients, and about 5 million people die from cancer every year worldwide. Lung cancer, liver cancer, gastric cancer, esophageal cancer, etc. have become the main cancers to be prevented and treated in China.

Chemotherapy is one of the main therapy among the conventional cancer treatment methods. Medicaments for chemotherapy such as Cisplatin can non-specifically block cell division and thus cause cell death, and they may also destroy the growth of normal cells while killing the tumor cells, thereby causing various adverse reactions and developing drug resistance.

Epidermal growth factor receptor (EGFR) is a receptor for epidermal growth factor (EGF) cell proliferation and signaling. EGFR belongs to a family of protein tyrosine kinases, and includes EGFR (Erb-B 1), Erb-B2 (HER-2/neu), Erb-B3 (Her3), and Erb-B4 (Her4). EGFR is located on a surface of cell membrane, and may form homodimers with ligands (such as EGF or TGFα) or heterodimers with other receptors in the family (such as Erb-B2, Erb-B3, or Erb-B4). Upon dimerization, EGFR may cause phosphorylation of key tyrosine residues in EGFR cells, thereby activating multiple downstream signaling pathways in the cell, including MAPK, Akt, and JNK pathways. These signaling pathways play an important role in cell proliferation, invasion, metastasis, and apoptosis.

It has been verified that overexpression and mutation of EGFR may lead to uncontrollable cell growth and are associated with the occurrence and progression of most cancers, such as lung cancer, colon cancer, breast cancer, etc. Therefore, EGFR has become an important target for the development of anticancer medicaments.

Existing small molecule inhibitors targeting EGFR can be divided into three generations:

(1) The first-generation small molecule EGFR inhibitors, including Gefitinib and Erlotinib, have shown good efficacy in the treatment of lung cancer and have been used as first-line drugs for the treatment of non-small cell lung cancer with EGFR activating mutations. Compared with chemotherapy, the first-generation small molecule EGFR inhibitors have the advantage of not producing side effects such as bone marrow suppression, nausea, and hair loss. However, after 10 to 12 months of treatment with the first-generation small molecule EGFR inhibitors such as Gefitinib, most patients still develop drug resistance. The drug resistance of most patients can be attributed to T790M mutation of EGFR gene, which reduces the affinity of drug to the target and causes tumor recurrence or disease progression.

(2) The second-generation small molecule EGFR inhibitors include Afatinib and others. Compared with the non-covalent reversible binding between the first-generation small molecule EGFR inhibitors and EGFR, the second-generation small molecule EGFR inhibitors inhibit the kinase activity of EGFR by forming a stable covalent bond with the kinase target. However, since these drugs also have an inhibitory effect on wild-type EGFR and other tyrosine protein kinases, the side effects are increased. At the same time, the second-generation EGFR inhibitors have low selectivity for the T790M mutation of EGFR gene, and thus cannot overcome tumor resistance.

(3) The third-generation small molecule EGFR inhibitors, represented by Osimertinib, can effectively and selectively against EGFR-TKI acquired T790M resistance, and can solve approximately 60% of the EGFR targeted drug resistance problems. However, accompanied with the use of third-generation small-molecule EGFR inhibitors, most patients also develop drug resistance.

Therefore, in order to solve the problems of drug resistance of small molecule EGFR inhibitors and improve the effectiveness of tumor treatment, it is urgent to provide novel compounds, especially those with novel skeletons, to address the problem of drug resistance in the treatment of EGFR inhibitors in tumors. Small cell lung cancer accounts for 15% to 20% of lung cancer, and it is more malignant with a poor prognosis. Due to the high heterogeneity of small cell lung cancer and the lack of molecularly targeted medicaments, the current clinical treatment is mainly based on a combination of cytotoxic chemotherapy medicaments and radiotherapy. Only 25% of patients with early-stage small cell lung cancer can achieve long-term disease control through concurrent chemoradiotherapy. However, for most patients with small cell lung cancer, the response to concurrent chemoradiotherapy is transient, with a median survival of less than 2 years for patients with early-stage small cell lung cancer and approximately 1 year for patients with metastatic small cell lung cancer. Currently, the use of immune checkpoint inhibitors (PD-1/PD-L1 antibodies) can benefit some patients with small cell lung cancer. Nevertheless, the high cost of biological medicaments has greatly increased patients' financial burden. Therefore, to improve the effectiveness of treatment and prolong the survival of patients with small cell lung cancer, it is urgent to develop small molecule compounds with a novel skeleton.

### SUMMARY

An object of the present disclosure is to provide a pyrimidine-2,4-diamine derivative represented by Formula 1, or a racemate, tautomer, enantiomer, diastereomer, isotope-substituted compound, prodrug or pharmaceutically acceptable salt thereof, 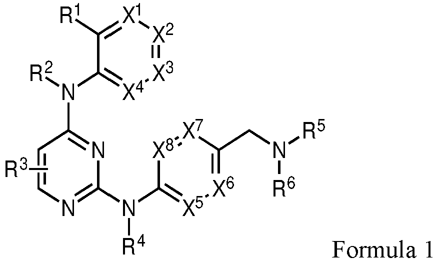 wherein:
X¹, X², X³, X⁴, X⁵, X⁶, X⁷, or X⁸ is each independently selected from N or CR⁷;
R¹ is selected from 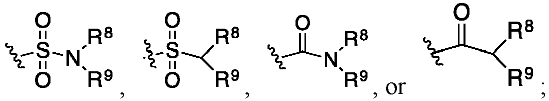
R² is selected from hydrogen, alkyl, or substituted alkyl;
R³ is selected from hydrogen, halogen, C₁ to C₆ alkyl, amino, (C₁ to C₆ alkyl)ₘ amino, (C₁ to C₆ alkyl)ₘ aminoalkyl, (C₁ to C₆ alkyl)ₘ amido, or (C₁ to C₆ alkyl)ₘ aminoacyl;
R⁴ is selected from hydrogen, alkyl, or substituted alkyl;
R⁵ and R⁶, together with N to which R⁵ and R⁶ are commonly bonded, form a 5-membered or 6-membered heterocyclic ring or a bridged ring; or R⁵ and R⁶, together with 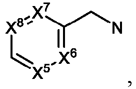 form a condensed ring;
the 5-membered or 6-membered heterocyclic ring is selected from 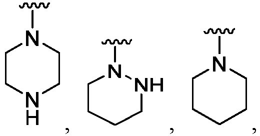 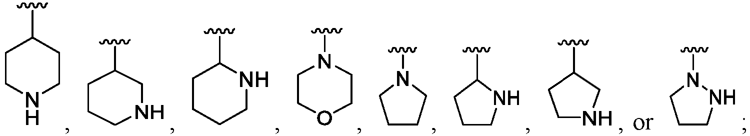 the bridged ring is selected from 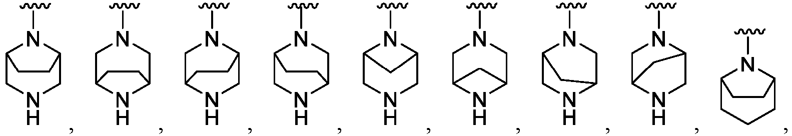 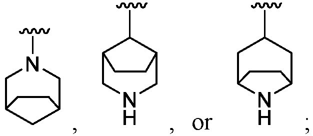 the condensed ring is selected from 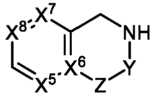 or 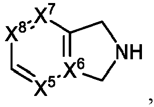 wherein Z or Y is each independently selected from a bond, (CH₂)ₙ, or NH;
the 5-membered or 6-membered heterocyclic ring, the bridged ring, or the condensed ring is each independently optionally substituted with one or more R¹⁰;
R⁸ and R⁹ are each independently selected from hydrogen, C₁ to C₆ alkyl, or C₃ to C₆ cycloalkyl;
R⁷ or R¹⁰ is each independently selected from hydrogen, C₁ to C₆ alkyl, halogenated C₁ to C₆ alkyl, hydroxyl C₁ to C₆ alkyl, C₁ to C₆ alkoxy, halogenated C₁ to C₆ alkoxy, halogen, cyano, hydroxyl, carboxyl, nitro, (C₁ to C₆ alkyl)ₘ aminocarbonyl, C₁ to C₆ alkylcarbonyl, (C₁ to C₆ alkyl)ₘ amino, (C₁ to C₆ alkyl)ₘ aminoalkyl, C₁ to C₆ alkoxyalkyl, (C₁ to C₆ alkyl)ₘ aminocarbonyl amino, sulfonic acid, C₁ to C₆ alkylsulfonyl, (C₁ to C₆ alkyl)ₘ aminosulfonyl, C₁ to C₆ alkylsulfonyl amino, or C₃ to C₆ cycloalkyl;
m is independently selected from 0, 1, or 2; and
n is 1 or 2.

In some embodiments, R¹ is 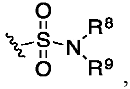 and preferably, R⁸ and R⁹ are each independently selected from H or methyl; preferably, R² is hydrogen; and preferably, R⁴ is hydrogen.

In some embodiments, R³ is halogen, and preferably, R³ is chlorine.

In some embodiments, the 5-membered or 6-membered heterocyclic ring is 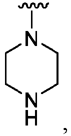 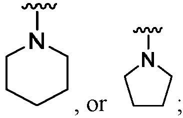 the bridged ring is 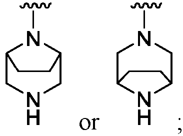 and R¹⁰ is selected from C₁ to C₄ alkyl or (C₁ to C₆ alkyl)ₘ amino, wherein m in R¹⁰ is 0, 1, or 2, and preferably, R¹⁰ is methyl or *N,N-*dimethylamino.

In some embodiments, X¹, X², X³, X⁴, X⁵, X⁶, X⁷, or X⁸ is CH; or only one of X¹, X², X³, and X⁴ is N and/or only one of X⁵, X⁶, X⁷, and X⁸ is N.

In some embodiments, the pyrimidine-2,4-diamine derivative is a compound represented by Formula 2, 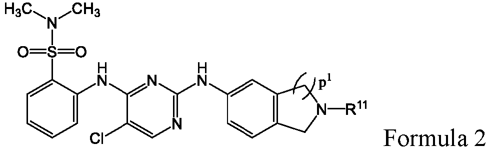
wherein R¹¹ is selected from C₁ to C₆ alkyl, hydroxyl C₁ to C₆ alkyl, C₁ to C₆ alkylcarbonyl, (C₁ to C₆ alkyl)ₘ aminoalkyl, or C₁ to C₆ alkoxyalkyl; and preferably, R¹¹ is selected from *N,N-*dimethylaminoethyl, methoxyethyl, and N-methylaminoethyl;
m is selected from 0, 1, or 2; and
p¹ is selected from 0, 1, 2, or 3.

In some embodiments, the pyrimidine-2,4-diamine derivative is a compound represented by Formula 3, 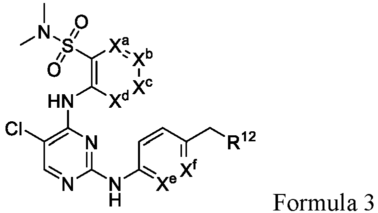 wherein:
X^{a}, X^{b}, X^{c}, X^{d}, X^{e}, or X^{f} is each independently selected from N or CH; or only one of X^{a}, X^{b}, X^{c}, and X^{d} is N and/or only one of X^{e} and X^{f} is N; and
R¹² is selected from 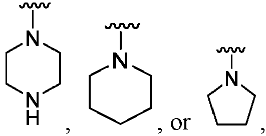 wherein R¹² is arbitrarily substituted with one or more substitutes of methyl, dimethylamine, or trimethylamine.

In some embodiments, the pyrimidine-2,4-diamine derivative is a compound represented by Formula 4, 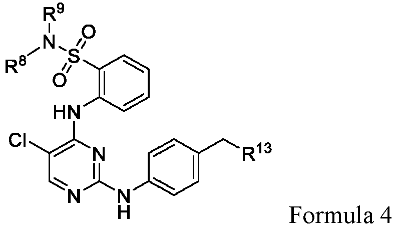 wherein:
R⁸ and R⁹ are each independently selected from hydrogen, C₁ to C₆ alkyl, or C₃ to C₆ cycloalkyl; and
R¹³ is selected from 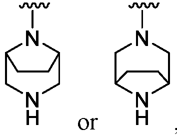 , wherein R¹³ is arbitrarily substituted with one or more substitutes of methyl.

In some embodiments, the pyrimidine-2,4-diamine derivative represented by Formula 1, or the racemate, tautomer, enantiomer, diastereomer, isotope-substituted compound, prodrug or pharmaceutically acceptable salt thereof include, but are not limited to, the following compounds:

| **Compound Number** | **Structure** | **Name** |
|---|---|---|
| **1** | 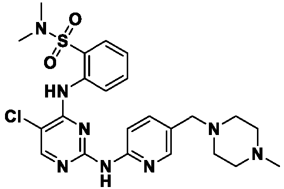 | 2-((5-chloro-2-((5-((4-methylpiperazin-1-yl)methyl)pyridin-2-yl)amino)pyrimidin-4-yl)amino)-*N,N-*dimethylbenzenesulfonamide |
| **2** | 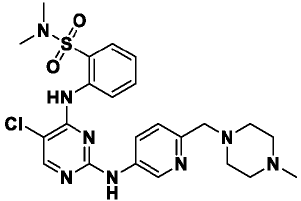 | 2-((5-chloro-2-((6-((4-methylpiperazin-1-yl)methyl)pyridin-3-yl)amino)pyrimidin-4-yl)amino)-*N,N-*dimethylbenzenesulfonamide |
| **3** | 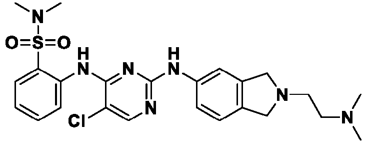 | 2-((5-chloro-2-((2-(2-(dimethylamino)ethyl)isodihydroindol-5-yl)amino)pyrimidin-4-yl)amino)-*N,N-*dimethylbenzenesulfonamide |
| **4** | 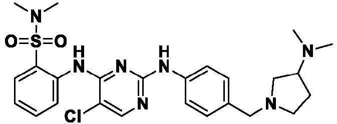 | 2-((5-chloro-2-((4-((3-(dimethylamino)pyrrolidin-1-yl)methyl)phenyl)amino)pyrimidin-4-yl)amino)-*N,N-*dimethylbenzenesulfonamide |
| **5** | 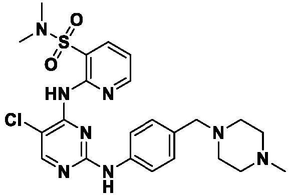 | 2-((5 -chloro-2-((4-((4-methylpiperazin-1-yl)methyl)phenyl)amino)pyrimidin-4-yl)amino)-*N,N*-dimethylpyridine-3-sulfonamide |
| **6** | 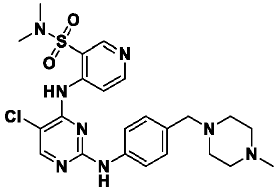 | 4-((5 -chloro-2-((4-((4-methylpiperazin-1-yl)methyl)phenyl)amino)pyrimidin-4-yl)amino)-*N,N*-dimethylpyridine-3-sulfonamide |
| **7** | 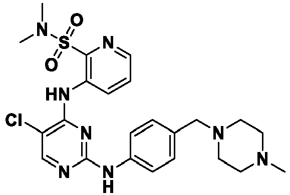 | 3-((5-chloro-2-((4-((4-methylpiperazin-1-yl)methyl)phenyl)amino)pyrimidin-4-yl)amino)-*N,N*-dimethylpyridine-2-sulfonamide |
| **8** | 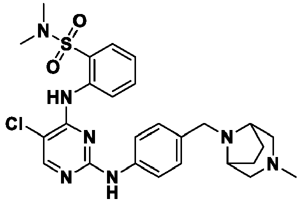 | 2-((5-chloro-2-((4-((3-methyl-3,8-diazabicyclo[3.2.1]octan-8-yl)methyl)phenyl)amino)pyrimidin-4-yl)amino)-*N,N-*dimethylbenzenesulfonamide |
| **9** | 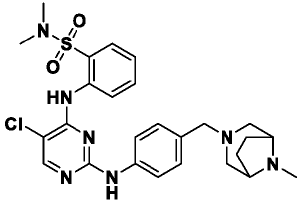 | 2-((5-chloro-2-((4-((8-methyl-3,8-diazabicyclo [3.2.1]octan-3-yl)methyl)phenyl)amino)pyrimidin-4-yl)amino)-*N,N-*dimethylbenzenesulfonamide |
| **10** | 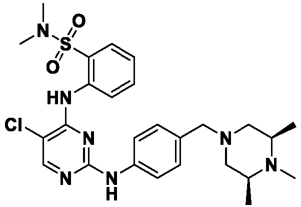 | 2-((5-chloro-2-((4-(((3R,5S)-3,4,5-trimethylpiperazin-1-yl)methyl)phenyl)amino)pyrimidin-4-yl)amino)-*N,N-*dimethylbenzenesulfonamide |
| **11** | 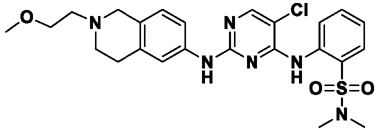 | 2-((5-chloro-2-((2-(2-methoxyethyl)-1,2,3,4-tetrahydroisoquinolin-6-yl)amino)pyrimidin-4-yl)amino)-*N,N-*dimethylbenzenesulfonamide |
| **12** | 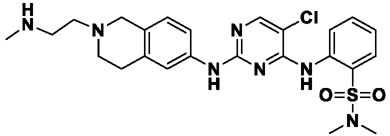 | 2-((5-chloro-2-((2-(2-(methylamino)ethyl)-1,2,3,4-tetrahydroisoquinolin-6-yl)amino)pyrimidin-4-yl)amino)-*N,N-*dimethylbenzenesulfonamide |
| **13** | 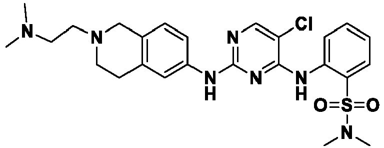 | 2-((5-chloro-2-((2-(2-(dimethylamino)ethyl)-1,2,3,4-tetrahydroisoquinolin-6-yl)amino)pyrimidin-4-yl)amino)-*N,N-*dimethylbenzenesulfonamide |
| **14** | 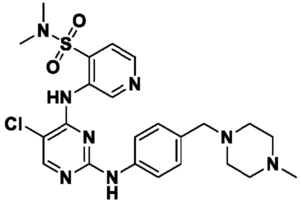 | 3-((5-chloro-2-((4-((4-methylpiperazin-1-yl)methyl)phenyl)amino)pyrimidin-4-yl)amino)-*N,N*-dimethylpyridine-4-sulfonamide |
| **15** | 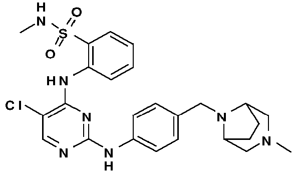 | 2-((5-chloro-2-((4-((3-methyl-3,8-diazabicyclo[3.2.1]octan-8-yl)methyl)phenyl)amino)pyrimidin-4-yl)amino)-N-methylbenzenesulfonamide |

Another aspect of the present disclosure provides a method for preparing the pyrimidine-2,4-diamine derivative represented by Formula 1, or the racemate, tautomer, enantiomer, diastereomer, isotope-substituted compound, prodrug or pharmaceutically acceptable salt thereof. The method includes: 1) performing a condensation reaction on an intermediate 1 and an intermediate 2 to generate an intermediate 3; and 2-1) performing a condensation reaction on the intermediate 3 and an intermediate 4 to generate the pyrimidine-2,4-diamine derivative represented by Formula 1; or 3-1) performing a condensation reaction on the intermediate 3 and an intermediate 5 to generate an intermediate 6, and 3-2) performing a condensation reaction on the intermediate 6 and an intermediate 7 to generate the pyrimidine-2,4-diamine derivative represented by Formula 1, according to the following reaction equation. 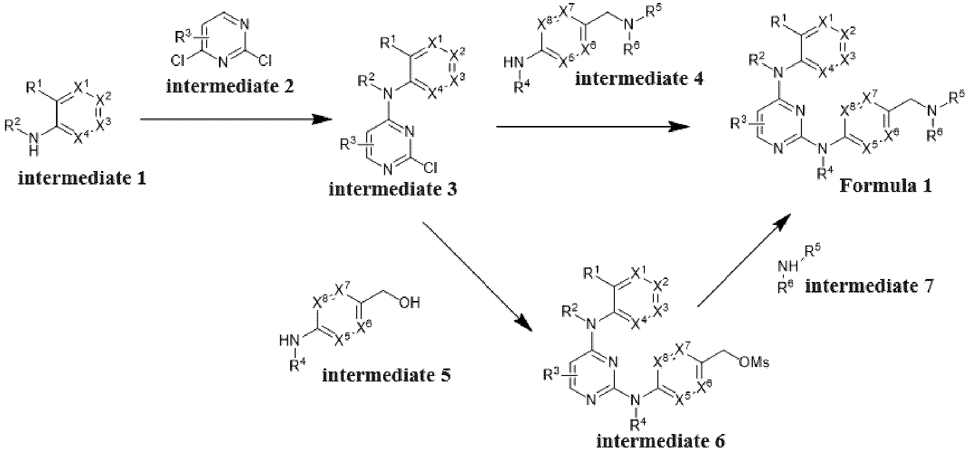 wherein X¹ to X⁸, R¹ to R⁶, m, and n are as defined above.

Another aspect of the present disclosure provides a pharmaceutical composition. The pharmaceutical composition includes: a therapeutically effective dose of a pyrimidine-2,4-diamine derivative, or a racemate, tautomer, enantiomer, diastereomer, isotope-substituted compound, prodrug or pharmaceutically acceptable salt thereof; and one or more pharmaceutically acceptable carriers or excipients.

The present disclosure further provides a method for preparing the above-mentioned pharmaceutical composition. The method includes mixing the pyrimidine-2,4-diamine derivative represented by formula 1, or the racemate, tautomer, enantiomer, diastereomer, isotope-substituted compound, prodrug or pharmaceutically acceptable salt thereof with the pharmaceutically acceptable carrier or excipient.

The present disclosure further provides use of the pyrimidine-2,4-diamine derivative represented by Formula 1, or the racemate, tautomer, enantiomer, diastereomer, isotope-substituted compound, prodrug or pharmaceutically acceptable salt thereof, or the pharmaceutical composition containing the same in the preparation of a medicament for treating a drug-resistant tumor.

The present disclosure further provides use of the pyrimidine-2,4-diamine derivative represented by Formula 1, or the racemate, tautomer, enantiomer, diastereomer, isotope-substituted compound, prodrug or pharmaceutically acceptable salt thereof, or the pharmaceutical composition containing the same for the treatment of a drug-resistant tumor.

In some embodiments, the drug-resistant tumor is an advanced solid tumor.

In some embodiments, the advanced solid tumor is advanced lung cancer, advanced thyroid cancer, advanced bladder cancer, advanced glioma, advanced pancreatic cancer, advanced melanoma, advanced gastric cancer, advanced colorectal cancer, advanced liver cancer, advanced cervical cancer, advanced ovarian cancer, or advanced esophageal cancer

In some embodiments, the advanced solid tumor is small cell lung cancer, or non-small cell lung cancer resistant to targeted drug therapy.

The present disclosure further provides a method for treating a drug-resistant tumor. The method includes: administering to a patient in need thereof a therapeutically effective dose of the pyrimidine-2,4-diamine derivative represented by Formula 1, or a racemate, tautomer, enantiomer, diastereomer, isotope-substituted compound, prodrug or pharmaceutically acceptable salt thereof, or the pharmaceutical composition containing the same.

In some embodiments, the drug-resistant tumor is an advanced solid tumor.

In some embodiments, the advanced solid tumor is advanced lung cancer, advanced thyroid cancer, advanced bladder cancer, advanced glioma, advanced pancreatic cancer, advanced melanoma, advanced gastric cancer, advanced colorectal cancer, advanced liver cancer, advanced cervical cancer, advanced ovarian cancer, or advanced esophageal cancer.

In some embodiments, the advanced solid tumor is small cell lung cancer or non-small cell lung cancer resistant to targeted drug therapy. The pyrimidine-2,4-diamine derivative represented by Formula 1, or the racemate, tautomer, enantiomer, diastereomer, isotope-substituted compound, prodrug or pharmaceutically acceptable salt thereof, as an active ingredient, may be prepared into a form suitable for administration by any appropriate route. The active ingredient is preferably in the form of a unit dose, or in a form that a patient can self-administer in a single dose. The unit dose of the active ingredient or composition of the present disclosure may be presented in the form of tablets, capsules, granules, pastes, lozenges, suppositories, or liquid formulations.

The dosage of the compound or composition used in the treatment methods according to the present disclosure may generally vary with the weight of the patient, the severity of the disease, and the relative efficacy of the compound. A general unit dosage of the compound of the present disclosure ranges from 0.1 mg to 1000 mg.

The pharmaceutical composition of the present disclosure may contain one or more carriers or excipients in addition to the active ingredient. The carrier or excipient can be selected from the following ingredients: fillers, disintegrants, binders, wetting agents, or lubricants. According to the administration method, a content of the active ingredient in the pharmaceutical composition may range from 0.1 wt% to 99 wt%.

The pharmaceutical composition containing the active ingredient may be in a form suitable for oral administration, such as tablets, capsules, granules, powders, suspensions, or syrups. Oral compositions may be prepared according to any method known in the art. The tablets, capsules, granules, and powders in the oral composition include active ingredients and pharmaceutically acceptable excipients suitable for preparing the above-mentioned oral composition for mixing. These excipients may be fillers, disintegrants, binders, and lubricants; and the oral composition may further contain the following carriers: sweeteners, flavoring agents, colorants, and preservatives to improve the taste and enhance stability.

Suspensions contain the active material and an excipient suitable for preparing the suspension. Such excipients include suspending agents, dispersing agents, and wetting agents. The suspension may also contain preservatives, colorants, or flavoring agents, etc.

The pharmaceutical composition of the present disclosure may be in the form of a sterile injection aqueous solution. Excipients may include solvents such as water, glycerol, sodium chloride, preservatives, solubilizers, etc.

The pharmaceutical composition of the present disclosure may be administered in the form of suppository for rectal administration. The excipients included in the suppository include a base, a thickener, an antioxidant, a hardening agent, etc.

### Definitions and general terms

Embodiments of the present disclosure are now described in detail, examples of which are illustrated by the accompanying structural and chemical formulas. The present disclosure is intended to cover all alternatives, modifications, and equivalent technical solutions, which all fall within the scope of the present disclosure as defined in the claims. One skilled in the art will recognize that many methods and materials similar or equivalent to those described herein may be used to practice the present disclosure. The present disclosure is not limited to the methods and materials described herein. In the event that one or more of the incorporated literature, patents, and similar materials differs from or contradicts this application, including, but not limited to, defined terms, term usage, described techniques, or the like, this application prevails.

Unless stated otherwise, the terms used in the specification and claims have the following meanings.

The term "DCM" refers to dichloromethane.

The term "TEA" refers to triethylamine.

The term "THF" means tetrahydrofuran.

The term "MeOH" refers to methanol.

The term "Pd/C" refers to palladium carbon.

The term "DMAC" refers to *N,N-*dimethylacetamide.

The term "NaH" refers to sodium hydride.

The term "AcOH" refers to acetic acid.

The term "NaBH(AcO)₃" refers to sodium triacetoxyborohydride.

The term "LiHMDS" refers to lithium bis(trimethylsilyl)amine.

The term "Pd₂(dba)₃" refers to trisdibenzylideneacetone dipalladium.

The term "t-BuONa" refers to sodium tert-butoxide.

The term "BINAP" refers to 1,1'-binaphthyl-2,2'-bisdiphenylphosphine.

The term "dioxane" refers to 1,4-dioxane.

The term "LiAlH₄" refers to lithium aluminum hydride.

The term "DCE" means dichloroethane.

The term "MeI" refers to methyl iodide.

The term "DMF" refers to *N,N-*dimethylformamide.

The term "K₂CO₃" refers to potassium carbonate.

The term "EtOAc" refers to ethyl acetate.

The term "EtOH" refers to ethanol.

The term "DCDMH" refers to 1,3-dichloro-5,5-dimethylhydantoin.

The term "NH₄Cl" refers to ammonium chloride.

The term "MsCl" means methanesulfonyl chloride.

The term "MeCN" refers to acetonitrile.

The term "con. HCl" means concentrated hydrochloric acid.

The term "(COCl)₂" refers to oxalyl chloride.

The term "BH₃" refers to borane.

The term "con. H₂SO₄" refers to concentrated sulfuric acid.

The term "(CH₂O)ₙ" refers to paraformaldehyde.

The term "Boc₂O" refers to di-tert-butyl dicarbonate.

The term "t-BuOK" refers to potassium tert-butoxide.

The term "CCl₄" refers to carbon tetrachloride.

The term "Cl₂" refers to chlorine gas.

The term "Br₂" refers to bromine.

The term "Cs₂CO₃" refers to cesium carbonate.

The term "X-Phos" refers to 2-dicyclohexylphosphino-2',4',6'-triisopropylbiphenyl.

The term "Pd(OAc)₂" refers to palladium acetate.

The term "TFA" refers to trifluoroacetic acid.

The term "PhMeSH" refers to benzylthiol.

The term "alkyl" refers to a straight or branched saturated hydrocarbon group. Non-limiting examples of alkyl include methyl, ethyl, propyl, isopropyl, n-butyl, isobutyl, tert-butyl, n-pentyl, 1,1-dimethylpropyl, 1,2-dimethylpropyl, 1-ethylpropyl, 2-methylbutyl, 3-methylbutyl, n-hexyl, and 1-ethyl-2-methylpropyl.

The term "alkoxy" refers to a group having a "W-O-" structure, in which W is an alkyl group. Non-limiting examples of alkoxy include methoxy, ethoxy, propoxy, isopropoxy, or tert-butoxy.

The term "halogenated alkyl" refers to alkyl substituted with one or more halogens. Non-limiting examples of halogenated alkyl include trifluoromethyl, trifluoroethyl, or trifluoropropyl, etc.

The term "halogenated alkoxy" refers to a group having a structure of "W-O-" and substituted with one or more halogens, in which W is an alkyl group. Non-limiting examples of the halogenated alkoxy group include trifluoromethoxy, trifluoroethoxy, or trifluoropropoxy.

The term "halogen" refers to fluorine, chlorine, bromine, or iodine.

The term "hydroxyalkyl" refers to alkyl substituted with one or more hydroxy groups. Non-limiting examples of hydroxyalkyl groups include hydroxymethyl or hydroxyethyl groups.

The term "cyano" refers to -CN.

The term "hydroxy" refers to -OH.

The term "carboxyl" refers to -COOH.

The term "nitro" refers to -NO₂.

The term "(C₁ to C₆ alkyl)ₘ aminocarbonyl" refers to a group having a structure of "NH₂CO-", "W-NHCO-", or "W₂-NCO-", in which W is an alkyl group. Non-limiting examples of (C₁ to C₆ alkyl)ₘ aminocarbonyl include methyl aminocarbonyl, ethyl aminocarbonyl, or dimethyl aminocarbonyl, etc.

The term "C₁ to C₆ alkylcarbonyl" refers to a group having a structure of "W-CO-", in which W is an alkyl group. Non-limiting examples of the C₁ to C₆ alkylcarbonyl include methyl carbonyl, ethyl carbonyl, or propyl carbonyl, etc.

The term "(C₁ to C₆ alkyl)ₘ amino" refers to a group having a structure of "NH₂-", "W-NH-", or "W₂-N-", in which W is an alkyl group. Non-limiting examples of (C₁ to C₆ alkyl)ₘ amino include methyl amino, ethyl amino, or dimethyl amino, etc.

The term "(C₁ to C₆ alkyl)ₘ aminoalkyl" refers to a group having a structure of "NH₂-W-", "W-NH-W-", or "W₂-N-W-", in which W is an alkyl group. Non-limiting examples of (C₁ to C₆ alkyl)ₘ aminoalkyl include methylaminomethyl, ethylaminomethyl, or dimethylaminomethyl, etc.

The term "C₁ to C₆ alkoxyalkyl" refers to a group having a structure of "W-O-W-", in which W is an alkyl group. Non-limiting examples of the C₁ to C₆ alkoxyalkyl include methoxymethyl, ethoxymethyl, etc.

The term "(C₁ to C₆ alkyl)ₘ aminocarbonyl amino" refers to a group having a structure of "NH₂CONH-", "W-NHCONH-", or "W₂-NCONH-", in which W is an alkyl group. Non-limiting examples of (C₁ to C₆ alkyl)ₘ aminocarbonyl amino include carbamido, methylaminocarbonyl amino, ethylaminocarbonyl amino, or dimethylaminocarbonyl amino, etc.

The term "sulfonic acid" refers to -SO₃OH.

The term "C₁ to C₆ alkylsulfonyl" refers to a group having a structure of "W-SO₂-", in which W is an alkyl group. Non-limiting examples of the C₁ to C₆ alkylsulfonyl include methylsulfonyl or ethylsulfonyl, etc.

The term "(C₁ to C₆ alkyl)ₘ aminosulfonyl" refers to a group having a structure of "NH₂SO₂-", "W-NH-SO₂-", or W₂-N-SO₂-", where W is an alkyl group. Non-limiting examples of (C₁ to C₆ alkyl)ₘ aminosulfonyl include methylaminosulfonyl, ethylaminosulfonyl, or dimethylaminosulfonyl, etc.

The term "C₁ to C₆ alkylsulfonyl amino" refers to a group having a structure of "W-SO₂-NH-", in which W is an alkyl group. Non-limiting examples of the C₁ to C₆ alkylsulfonyl amino include methylsulfonyl amino or ethylsulfonyl amino, etc.

The term "cycloalkyl" refers to a saturated or partially saturated cyclic hydrocarbon group. The cycloalkyl group consists of 3 to 15 carbon atoms, such as 3 to 6 carbon atoms. Non-limiting examples of the cycloalkyl group include cyclopropyl, cyclobutyl, cyclopentyl, or cyclohexyl, etc.

The term "substituted" means that one or more, preferably 1 to 3, hydrogen atoms in a group are independently substituted with a corresponding number of substituents. Substituents are merely presented at their possible chemical positions, and one skilled in the art is able to (either experimentally or theoretically) determine the possible or impossible substitutions without undue effort.

The term "stereoisomer" is a compound having the same chemical configuration, but with different spatial arrangements of atoms or groups. Stereoisomers include enantiomers, diastereomers, conformational isomers (rotamers), geometric isomers (cis/trans) isomers, atropisomers, etc.

The term "chiral" is a molecule that has the property of not overlapping with its mirror image, while "achiral" is a molecule that can overlap with its mirror image.

The term "enantiomer" means two isomers of a compound that cannot overlap each other but mutually mirror.

The term "diastereomers" refer to stereoisomers, which have two or more centers of chirality, and whose molecules do not mirror each other. The diastereomers have different physical properties, such as melting points, boiling points, spectral properties, and reactivity. A mixture of diastereomers may be separated by high resolution analytical procedures such as electrophoresis and chromatography, for example HPLC.

Many organic compounds exist in optically active forms, i.e., they are capable of rotating a plane of plane-polarized light. When describing optically active compounds, the prefixes *D* and *L*, or *R* and *S* are used to denote the absolute configurations of the molecule with respect to one or more chiral centers. The prefixes D and L, or (+) and (-) are symbols used to specify a rotation of plane-polarized light caused by a compound, where (-) or L indicates that the compound is levorotatory, and the prefix (+) or D indicates that the compound is dextrorotatory. A specific stereoisomers may be an enantiomer, and a mixture of such isomers is called an enantiomeric mixture. A mixture of enantiomers in 50:50 is called a racemic mixture or a racemate, which may occur when there is no stereoselectivity or stereospecificity in a chemical reaction or process.

Any asymmetric atom (e.g., carbon, etc.) of the compounds of the present disclosure may be present in a racemic or enantiomerically enriched form, e.g., in the (R)-, (S)-, or (R,S)-configuration. In some embodiments, each asymmetric atom has at least a 50% enantiomeric excess, at least a 60% enantiomeric excess, at least a 70% enantiomeric excess, at least an 80% enantiomeric excess, at least a 90% enantiomeric excess, at least a 95% enantiomeric excess, or at least a 99% enantiomeric excess with respect to the (R)- or (S)-configuration.

In accordance with the selection of raw materials and methods, the compound of the present disclosure may exist in the form of one of the possible isomers or a mixture thereof, for example, such as racemic isomer and diastereoisomeric mixture, depending on the number of asymmetric carbon atoms. The optically active (*R*)- or (*S*)-isomer can be prepared using chiral synthons or chiral preparations, or resolved using conventional techniques. If the compound contains a double bond, the substituents may be in *E*- or *Z*-configuration; if the compound contains a disubstituted cycloalkyl, the substituent of the cycloalkyl may has a *cis-* or *trans-*conformation.

Any resulting mixture of stereoisomers may be separated into the pure or substantially pure geometric isomers, enantiomers, diastereomers, based on differences in the physicochemical properties of the components, for example, by chromatography and/or fractional crystallization.

Any racemates of the end products or intermediates obtained may be resolved into the optical antipodes by a known method familiar to those skilled in the art, for example, by separation of the obtained diastereoisomeric salts thereof. The racemic products may also be separated by chiral chromatography, such as high performance liquid chromatography (HPLC) using a chiral adsorbent. In particular, enantiomers may be prepared by asymmetric synthesis, for example, see Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers of different energies that are interconvertible via a low energy barrier. If tautomerism is possible (such as in solution), a chemical equilibrium of the tautomer may be achieved. For example, proton tautomer (also known as prototropic tautomer) include interconversions via migration of a proton, such as keto-enol and imine-enamine isomerization.

As described herein, the compounds of the present disclosure may be optionally substituted with one or more substituents, such as the above general formula compounds, or as specific examples, and subclasses in the embodiments, and classes of compounds included in the present disclosure. It can be understood that the term "optionally substituted" may be used interchangeably with the term "substituted or unsubstituted." In general, the term "optionally," whether preceded by the term "substituted" or not, means that one or more hydrogen atoms in a given structure are substituted with the radical of a specified substituent. Unless otherwise indicated, an optionally substituted group may have a substituent at each substitutable position of the group. When more than one position in a given formula may be substituted with one or more substituents selected from a specified group, the substituents may be the same or different at each position. The substituents may be, but are not limited to, deuterium, hydroxyl, amino, halogen, cyano, aryl, heteroaryl, alkoxy, alkylamino, alkylthio, alkyl, alkenyl, alkynyl, heterocyclic, sulfhydryl, nitro, aryloxy, heteroaryloxy, oxo (=O), carboxyl, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂₋, carboxyl-substituted alkoxy, etc.

The term "isotope-substituted compound" refers to a compound in which a hydrogen atom is substituted with deuterium or tritium, or a carbon atom is substituted with ¹³C, ¹⁴C, or ¹⁵C, or an N atom is substituted with ¹³N, ¹⁵N, ¹⁶N, or an oxygen atom is substituted with ¹⁵O or ¹⁷O, or a fluorine atom is substituted with ¹⁷F or ¹⁹F, or an iodine atom is substituted with ¹²⁸I in the group.

In addition, it should be noted that, unless otherwise clearly stated, the description method used in the present disclosure "each of...independently", "... each independently", and"... independently" may be interchanged and should be understood in a broad sense, indicating that the specific options expressed by the same symbols in different groups do not affect each other, or that the specific options expressed by the same symbols in the same group do not affect each other. As an example, the specific options of R in the groups of -RC(=O)-, - NRC(S)NR'-, -NRC(O)O-, -C(O)NR-, or -NR-C(O)- are not affected by each other; at the same time, if two or more R appear in the same chemical formula, the specific options of R are not affected by each other.

The term "pharmaceutical composition" means a composition containing one or more of the compounds described herein or mixture with other chemical components, as well as other components such as pharmaceutically acceptable carriers and excipients. The purpose of a pharmaceutical composition is to facilitate administration to an organism, facilitate the absorption of the active ingredients, and thereby exert biological activity.

Pharmaceutical compositions suitable for the present disclosure include a plurality of compositions containing an effective amount of active ingredients to achieve their intended purpose. Determination of such multiple effective amounts is well within the capability of those skilled in the art, in particular in light of the detailed disclosure provided herein. Generally, the plurality of compounds according to the present disclosure are effective over a wide dosage range. For example, in the treatment of adults, dosages ranging from 0.01 milligrams to 1,000 milligrams (mg) per day, 0.5 mg to 100 mg per day, 1 mg to 50 mg per day, and 5 mg to 40 mg per day are examples of dosages that may be used. A non-limiting dosage ranges from 10 mg to 30 mg per day. The exact dose may depend on the route of administration, the administration form of the compound, the subject to be treated, the body weight of the subject to be treated, the bioavailability, adsorption, distribution, metabolism of the compound(s), excretion (ADME) toxicity of the compound(s), and the preference and experience of the attending physician.

The term "pharmaceutically acceptable salt" refers to a salt of the compounds of the present disclosure, which are safe and effective when used in mammals and have the desired biological activity.

The term "pharmaceutically acceptable salt" refers to an organic and inorganic salt of the compound of the present disclosure. Pharmaceutically acceptable salts are well known in the art, such as recorded in S.M. Berge et al., J. Pharmaceutical Sciences, 66, 1-19, 1977. Non-toxic acid form of the pharmaceutically acceptable salt includes, but is not limited to, inorganic acid salts formed by reaction with amino groups, such as hydrochlorides, hydrobromides, phosphates, sulfates, perchlorates, and organic acid salts, such as acetates, oxalates, maleates, tartrates, citrates, succinates, malonates, or salts obtained by other methods described in the literature, such as ion exchange method. Other pharmaceutically acceptable salts include adipate, alginate, ascorbate, aspartate, benzenesulfonate, benzoate, bisulfate, borate, butyrate, camphorate, camphorsulfonate, cyclopentylpropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, etc. Salts derived from appropriate bases include alkali metal, alkaline earth metal, ammonium, and salts of N+(C_{1 to 4} alkyl)₄. The present disclosure also contemplates quaternary ammonium salts formed by any compound containing a N group. Water-soluble, or oil-soluble, or dispersible products may be obtained by quaternization. Alkali metal or alkaline earth metal salts include sodium, lithium, potassium, calcium, magnesium, etc. Pharmaceutically acceptable salts further include suitable, non-toxic ammonium/quaternary ammonium salts and amine cations resistant to the formation of equilibrium ions, such as halides, hydroxides, carboxylates, sulfates, phosphates, nitrates, C_{1 to 8} sulfonates, and aromatic sulfonates.

A plurality of pharmaceutically acceptable salts is generally known to those of ordinary skill in the art, and includes salts of the active compounds prepared with relatively nontoxic acids or bases, depending on the specific substituents present on the compounds described herein. When compounds of the present disclosure contain relatively acidic functional groups, base addition salts may be obtained by contacting the neutral form of the compounds with a sufficient amount of the desired base, either neat or in a suitable inert solvent or by ion exchange, whereby one basic counterion (base) in an ionic complex is replaced by another. Examples of pharmaceutically acceptable base addition salts include sodium, potassium, calcium, ammonium, organic amino or magnesium salts, or a similar salt.

The term "prodrug" refers to a compound that is converted in vivo to a compound represented by formula (X). Such conversion is affected by hydrolysis of the prodrug in the blood or by enzymatic conversion to the parent structure in the blood or tissues. The prodrug compounds of the present disclosure may be esters. In the prior art, esters that may be used as prodrugs include phenyl esters, aliphatic (C₁ to C₂₄) esters, acyloxymethyl esters, carbonates, carbamates, and amino acid esters. For example, if a compound of the present disclosure contains a hydroxy group, it may be acylated to obtain a prodrug form of the compound. Other prodrug forms include phosphate esters, such as those derived by phosphorylation of a hydroxyl group on the parent moiety. A complete discussion of prodrugs may be found in the following references: T. Higuchi and V. Stella, Pro-medicaments as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

As used herein, the term "treating" may include reversing, alleviating, inhibiting the development of the disease, preventing or reducing the disease or condition to which the term applies, or one or more symptoms or manifestations of the disease or condition.

The term "preventing" means not causing a disease, condition, symptom, or manifestation, or a worsening of severity. Thus, the multiple compounds of the present disclosure may be administered prophylactically to prevent or reduce the occurrence or recurrence of the disease or condition.

As used herein, the term "treating" any disease or disorder refers to all measures that can slow down, interrupt, prevent, control, or stop the progression of the disease or disorder, but does not necessarily mean that all symptoms of the disease or disorder disappear. It also includes preventive treatment of the symptoms, especially in patients who are prone to such diseases or disorders. In some of these embodiments, "treating" refers to improve the disease or condition (i.e., the progression of the disease or at least one clinical symptom thereof is slowed, or arrested, or alleviated). In other embodiments, "treating" refers to alleviating or ameliorating at least one physical parameter, including physical parameters that may not be noticeable to the patient. In other embodiments, "treating" refers to modulating the disease or condition physically (e.g., stabilization of observable symptoms) or physiologically (e.g., stabilization of a bodily parameter), or both. In other embodiments, "treating" refers to preventing or delaying the onset, occurrence, or worsening of a disease or condition.

As used herein, the term "therapeutically effective amount" or "therapeutically effective dose" refers to the amount of the compound of the present disclosure that can trigger a biological or medical response in an individual (e.g., reducing or inhibiting enzyme or protein activity, or alleviating symptoms, alleviating disorders, slowing or delaying disease progression, or preventing disease, etc.).

The pyrimidine-2,4-diamine derivatives according to the present disclosure have high inhibitory activity on advanced tumor cell lines, in particular on non-small cell lung cancer, and in particular on non-small cell lung cancer resistant to targeted drug therapy. It can be concluded that the pyrimidine-2,4-diamine derivatives according to the present disclosure may be used to treat advanced tumors, in particular non-small cell lung cancer, and in particular non-small cell lung cancer resistant to targeted drug therapy.

Additional aspects and advantages of the present disclosure will be given in part in the following descriptions, become apparent in part from the following descriptions, or be learned from the practice of the present disclosure.

### BRIEF DESCRIPTION OF THE DRAWINGS

The foregoing and/or additional aspects and advantages of the present disclosure will become apparent and more readily appreciated from the following descriptions made with reference to the accompanying drawings, in which:
FIG. 1 is a graph showing changes in tumor volume of mice after treatment with different medicaments according to an embodiment of the present disclosure; and
FIG. 2 is a graph showing changes in body weight of mice after treatment with different medicaments according to an embodiment of the present disclosure.

### DETAILED DESCRIPTION

The embodiments of the present disclosure are described in detail below. The embodiments described below are exemplary and are only used to explain the present disclosure, and are not to be construed as limiting the present disclosure.

Solutions of the present disclosure will be explained below in connection with the examples. It will be appreciated by those skilled in the art that the following examples are merely illustrative of the present disclosure and should not be construed as limiting the scope of the present disclosure. Techniques or conditions that are not specified in the examples shall be performed in accordance with the techniques or conditions described in the literatures in the art or in accordance with the product instruction. Reagents or instruments without indicating the manufacturer are all common products that are commercially available.

The present disclosure is further described below in conjunction with embodiments, but these embodiments are not intended to limit the scope of the present disclosure.

### Example 1

### Synthesis of 2-((5-chloro-2-((5-((4-methylpiperazin-1-yl)methyl)pyridin-2-yl)amino)pyrimidin-4-yl)amino)-N,N-dimethylbenzenesulfonamide (Compound 1)

### Step 1: Synthesis of 2-nitro-N,N-dimethylbenzenesulfonamide (1-2)

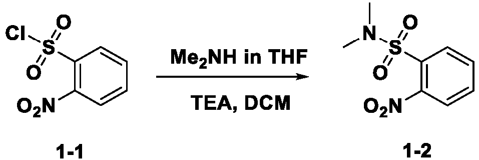

2-nitrobenzenesulfonyl chloride (1-1, 20.0 g, 90.5 mmol, 1.0 eq) was dissolved in 300 mL of DCM, and cooled down to 0°C. Then, TEA (37.0 g, 271 mmol, 3.0 eq) and 2 M solution of Me₂NH in THF (81.4 g, 135 mmol, 1.5 eq) were added dropwise, heated to 30°C, and reacted for 2 hours. When a completion of reaction was monitored by thin-layer chromatography (TLC), the reaction solution was concentrated. The system was diluted with 300 mL of water, extracted with DCM for three times (300 mL each time), and concentrated in vacuum to obtain a crude product. The crude product was slurried with 40.0 mL of methyl tert-butyl ether to obtain the target compound 1-2 (12.0 g) as a white solid with a yield of 57.6%.

LCMS: 231.0 ([M+H]⁺).

### Step 2: Synthesis of 2-amino-N,N-dimethylbenzenesulfonamide (1-3)

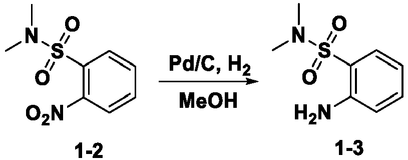

Compound 1-2 (12.0 g, 52.2 mmol) was dissolved in 84.0 mL of MeOH, 1.2 g of Pd/C was added thereto, and the mixture reacted at 25°C for 8 hours under hydrogen protection. The reaction solution was filtered and concentrated to obtain the target compound 1-3 (10.0 g), which was directly used in the next step of reaction.

LCMS: 201.2 ([M+H]⁺).

### Step 3: Synthesis of 2-((2,5-dichloropyrimidin-4-yl)amino)-N,N-dimethylbenzenesulfonamide (1-5)

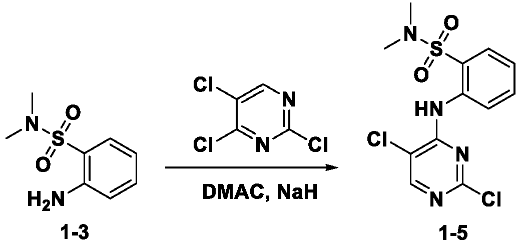

Compound 1-3 (10.0 g, 50.0 mol, 1.0 eq) was dissolved in 120 mL of DMAC, and NaH (6.00 g, 150 mmol, 3.0 eq) was added thereto at 25°C. After the addition was complete, the mixture reacted at room temperature for 2 hours. 20.0 mL of 2,4,5-trichloropyrimidine (27.2 g, 150 mmol, 3.0 eq) in DMAC was added to the reaction solution, and the mixture reacted at room temperature for 3 hours. 60 mL of water and 60 mL of methyl tert-butyl ether were added to the reaction system, and the mixture reacted at 25°C for 30 minutes. The reaction solution was then filtered and dried to obtain the target compound 1-5 (4.00 g) as a yellow solid with a yield of 31.2%.

¹H NMR (300 MHz, CDCl₃): δ 9.86 (m, 1H), 8.60 (dd, J = 8.4, 0.9 Hz, 1H), 8.28 (s, 1H), 7.86 (dd, J = 8.0, 1.6 Hz, 1H), 7.73 - 7.55 (m, 1H), 7.30 (dd, J = 11.4, 4.1 Hz, 1H), 2.75 (d, J = 6.8 Hz, 6H).

### Step 4: Synthesis of 1-((6-bromopyridin-3-yl)methyl)-4-methylpiperazine (1-7)

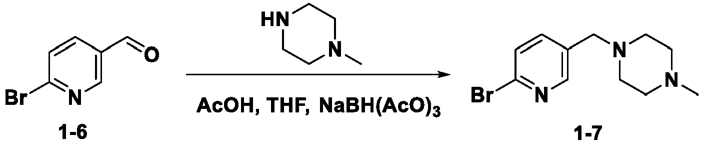

6-bromonicotinaldehyde (1-6, 2.00 g, 10.8 mmol, 1.0 eq) and 1-methylpiperazine (5.40 g, 54 mmol, 5.0 eq) were dissolved in 30 mL of THF. At 0°C, 0.3 g of AcOH was added thereto, and the mixture reacted for 30 minutes. NaBH(AcO)₃ (11.5 g, 54.0 mmol, 5.0 eq) was added to the system, the temperature was slowly raised to room temperature, and the mixture reacted overnight. The reaction solution was filtered and concentrated to obtain a crude product. The crude product was purified by silica gel column to obtain the target compound 1-7 (800 mg) as a white solid with a yield of 41.2%.

### Step 5: Synthesis of 5-((4-methylpiperazin-1-yl)methyl)pyridin-2-amine (1-9)

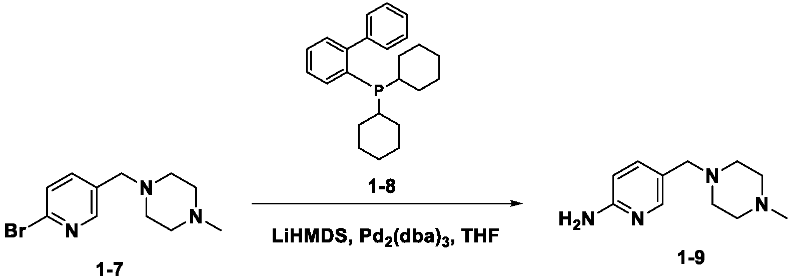

Compound 1-7 (0.5 g, 1.86 mmol, 1.0 eq) was dissolved in 5 mL of dry THF, and added with [1,1'-biphenyl]-2-yl dicyclohexylphosphane (1-8, 190 mg, 0.186 mmol, 0.1 eq), and the mixture reacted for 5 minutes. Then, LiHMDS (5.6 mL) and Pd₂(dba)₃ (75.7 mg, 0.093 mmol, 0.05 eq) were added to the suspension, and the temperature was raised to 65°C to react for 4 hours. 30 mL of DCM and 30 mL of water were added to the reaction solution. The system was adjusted to pH 2 with 1 N hydrochloric acid, and then extracted with DCM for 3 times, 30 mL each time. The aqueous phase was adjusted to pH 9 with 2 N sodium hydroxide solution, and then extracted with DCM for 3 times, 30 mL each time. The organic phases were combined and concentrated in vacuum to obtain the target compound 1-9 (210 mg) as a yellow solid.

LCMS: 207.2 ([M+H]⁺).

### Step 6: Synthesis of 2-((5-chloro-2-((5-((4-methylpiperazin-1-yl)methyl)pyridin-2-yl)amino)pyrimidin-4-yl)amino)-N,N-dimethylbenzenesulfonamide (Compound 1)

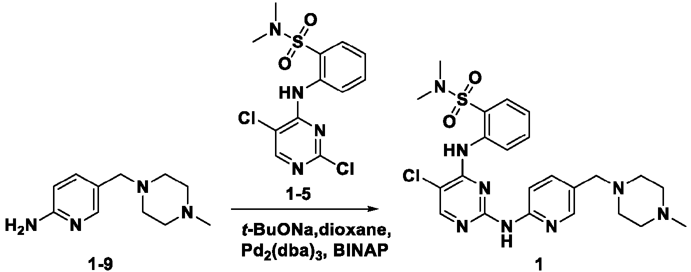

Compound 1-9 (180 mg, 0.87 mmol, 1.0 eq), compound 1-5 (360 mg, 0.96 mmol), and t-BuONa (40.0 mg, 1.09 mmol, 1.25 eq) were dissolved in 2.0 mL of dioxane and replaced with nitrogen for three times. Under nitrogen conditions, Pd₂(dba)₃ (90 mg, 0.087 mmol, 0.1 eq) and BINAP (108.3 mg, 0.174 mmol, 0.2 eq) were added. The reaction solution was heated to 130°C in a microwave reactor and reacted for 1.5 hours. 4.0 mL of water was added to the reaction solution, and the reaction solution was extracted with DCM for three times, 30 mL each time. The organic phases were combined, washed twice with saturated brine, 20 mL each time, and concentrated under reduced pressure to obtain a crude product. The crude product was purified by preparative liquid chromatography to obtain the target compound 1 (8.1 mg) as a white solid with a yield of 11.7%.

¹H NMR (400 MHz, DMSO-*d*₆): *δ* 9.97 (s, 1H), 9.52 (s, 1H), 8.91 (d, *J* = 8.4 Hz, 1H), 8.36 (s, 1H), 8.19 (d, *J* = 2.0 Hz, 1H), 7.94 (d, *J* = 8.4 Hz, 1H), 7.82 (dd, *J* = 8.0, 1.6 Hz, 1H), 7.76-7.67 (m, 1H), 7.58 (dd, *J=* 8.4, 2.0 Hz, 1H), 7.38-7.34 (m, 1H), 3.41 (s, 2H), 2.65 (s, 6H), 2.48-2.22 (m, 8H), 2.15 (s, 3H).

LCMS: 517.2 ([M+H]⁺).

### Example 2

### Synthesis of 2-((5-chloro-2-((6-((4-methylpiperazin-1-yl)methyl)pyridin-3-yl)amino)pyrimidin-4-yl)amino)-N,N-dimethylbenzenesulfonamide (Compound 2)

### Step 1: Synthesis of 2-(bromomethyl)-5-nitropyridine (2-1)

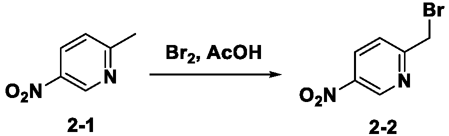

2-methyl-5-nitropyridine (2-1, 8.7 g, 63 mmol, 1.0 eq) was dissolved in AcOH (90 mL). Br₂ (5 g, 31.5 mmol, 0.5 eq) was added to the solution, the temperature was heated to 100°C, and the mixture reacted for 30 minutes. The reaction system was cooled to room temperature, diluted with 50 mL of water, and then extracted with methyl tert-butyl ether for three times, 100 mL each time. The organic phases were combined, washed twice with saturated sodium bicarbonate solution, 100 mL each time, then dried over anhydrous sodium sulfate, filtered, and concentrated to obtain a crude product. The crude product was purified by silica gel column (PE: EtOAc = 10: 1) to obtain the crude product 2-2 (8.5 g), which was directly used in the next step.

LCMS: 217.0, 219.0 ([M+H]⁺).

### Step 2: Synthesis of 1-methyl-4-((5-nitropyridin-2-yl)methyl)piperazine (2-3)

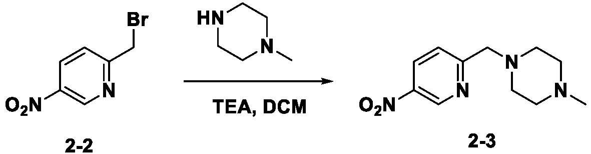

Compound 2-2 (8.5 g, 39 mmol, 1.0 eq) and 1-methylpiperazine (3.9 g, 39 mmol, 1.0 eq) were dissolved in DCM (80 mL) and added with TEA (5.9 g, 58.8 mmol, 2.0 eq), and the mixture reacted at room temperature for 12 hours. The reaction system was diluted with 100 mL of water, and then extracted with DCM for three times, 250 mL each time. The organic phases were combined, washed twice with saturated brine, 250 mL each time, then dried over anhydrous sodium sulfate, filtered, and concentrated to obtain a crude product. The crude product was purified by silica gel column (DCM: MeOH = 10: 1) to obtain the target compound 2-3 (2.5 g) as a brown solid. The yield in two-step reaction was 16.8%.

¹H NMR (300 MHz, CDCl₃): *δ* 9.34 (d, *J=* 2.4 Hz, 1H), 8.42 (dd, *J* = 8.7, 2.4 Hz, 1H), 7.66 (d, *J=* 8.7 Hz, 1H), 3.77 (s, 2H), 2.68-2.42 (m, 8H), 2.33 (s, 3H).

### Step 3: Synthesis of 6-((4-methylpiperazin-1-yl)methyl)pyridin-3-amine (2-4)

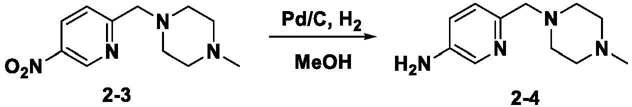

Compound 2-3 (2.5 g, 10.6 mmol, 1.0 eq) was dissolved in MeOH (25 mL), and then Pd/C (250 mg) was added to the solution. Under hydrogen protection, the mixture reacted at room temperature for 2 hours and filtered. The filtrate was concentrated to obtain the target compound 2-4 (2.0 g) as a brown solid with a yield of 91.6%.

LCMS: 207.2 ([M+H]⁺).

### Step 4: Synthesis of 2-((5-chloro-2-((6-((4-methylpiperazin-1-yl)methyl)pyridin-3-yl)amino)pyrimidin-4-yl)amino)-N,N-dimethylbenzenesulfonamide (Compound 2)

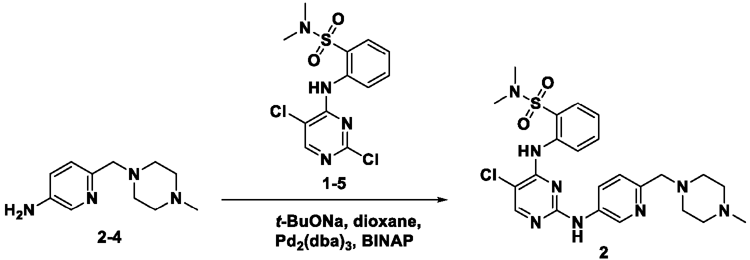

Compound 2-4 (80 mg, 0.39 mmol, 1.2 eq) and compound 1-5 (111 mg, 0.32 mmol, 1.0 eq) were dissolved in dioxane (5 mL), and BIANP (24.2 mg, 0.04 mmol, 0.1 eq), t-BuONa (46.6 mg, 0.49 mmol, 1.5 eq), and Pd₂(dba)₃ (17.7 mg, 0.02 mmol, 0.05 eq) were added thereto. Under nitrogen protection, the temperature was raised to 100°C, and the mixture reacted for 12 hours. The reaction system was cooled to room temperature, quenched by adding 15 mL of water, and then extracted with EtOAc for three times, 25 mL each time. The organic phases were combined, washed twice with saturated brine, 10 mL each time, then dried over anhydrous sodium sulfate, filtered, and concentrated to obtain a crude product. The crude product was purified by preparative thin layer chromatography (DCM: MeOH = 10: 1) to obtain the target compound 2 (19.4 mg) as an off-white solid with a yield of 9.7%.

¹H NMR (400 MHz, DMSO-*d*₆): *δ* 9.67 (s, 1H), 9.34 (s, 1H), 8.64 (d, *J* = 2.4 Hz, 1H), 8.56-8.51 (m, 1H), 8.31 (s, 1H), 8.03-7.99 (m, 1H), 7.83 (dd, *J=* 8.1, 1.5 Hz, 1H), 7.72-7.66 (m, 1H), 7.41-7.35 (m, 1H), 7.27 (d, *J=* 8.4 Hz, 1H), 3.51 (s, 2H), 2.65 (s, 6H), 2.46-2.24 (m, 8H), 2.16 (s, 3H).

LCMS: 517.1 [M+H]⁺.

### Example 3

### Synthesis of 2-((5-chloro-2-((2-(2-(dimethylamino)ethyl)isodihydroindol-5-yl)amino)pyrimidin-4-yl)amino)-N,N-dimethylbenzenesulfonamide (Compound 3)

### Step 1: Synthesis of 2-(2-(dimethylamino)ethyl)-5-nitroisodihydroindole-1,3-dione acetate (3-2)

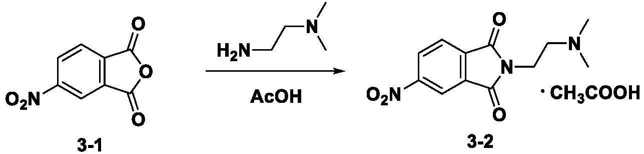

Compound 5-nitroisobenzofuran-1,3-dione (3-1, 11.1 g, 57.5 mmol, 1.0 eq) was dissolved in AcOH (75 mL). The compound *N,N*-dimethylethane-1,2-diamine (5.1 g, 57.5 mmol, 1.0 eq) was added thereto at room temperature. Then, the temperature was raised to 110°C, and the mixture reacted for 12 hours. The reaction solution was cooled to room temperature and then filtered, and the filtrate was concentrated to obtain a crude product. The crude product was slurried with MeOH (10 mL) and EtOAc (90 mL) to obtain the target compound 3-2 (20 g, crude) as a yellow solid.

LCMS: 264.1 ([M+H]⁺).

### Step 2: Synthesis of 5-amino-2-(2-(dimethylamino)ethyl)isodihydroindole-1,3-dione acetate (3-3)

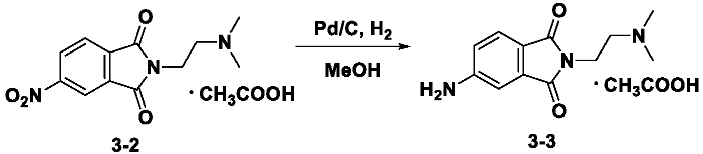

Compound 3-2 (20 g, crude product) was dissolved in MeOH (300 mL), Pd/C (2.0 g) was added thereto, and the mixture reacted at room temperature for 5 hours under hydrogen protection. The mixture was filtered, and the filtrate was concentrated to obtain the target compound 3-3 (12.4 g) as a yellow solid with a two-step reaction yield of 73.5%.

LCMS: 234.1 ([M+H]⁺).

### Step 3: Synthesis of 2-(2-(dimethylamino)ethyl)isodihydroindol-5-amine (3-4)

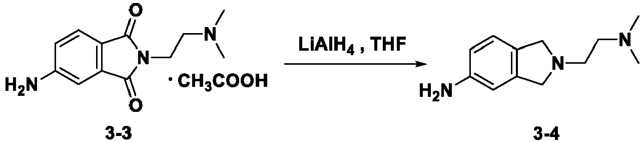

Compound 3-3 (1.0 g, 2.14 mmol, 1.0 eq) was dissolved in anhydrous THF (10 mL), and the temperature was cooled to 0°C. LiAlH₄ (407 mg, 1.07 mmol, 5.0 eq) was added in batches, and the reaction system was heated to 65°C to react for 24 hours. 1 mL of water, 1 mL of 15% sodium hydroxide solution, and 3 mL of water were added in sequence to the reaction solution for quench. Then, 4 g of sodium sulfate was added. After the addition was complete, the mixture reacted for 1 hour. The reaction system was filtered, and the filtrate was concentrated to obtain a crude product. The crude product was purified by silica gel column (DCM: MeOH = 10: 1) to obtain the target compound 3-4 (300 mg) as a black solid with a yield of 42.7%.

LCMS: 206.2 ([M+H]⁺).

### Step 4: Synthesis of 2-((5-chloro-2-((2-(2-(dimethylamino)ethyl)isodihydroindol-5-yl)amino)pyrimidin-4-yl)amino)-N,N-dimethylbenzenesulfonamide (Compound 3)

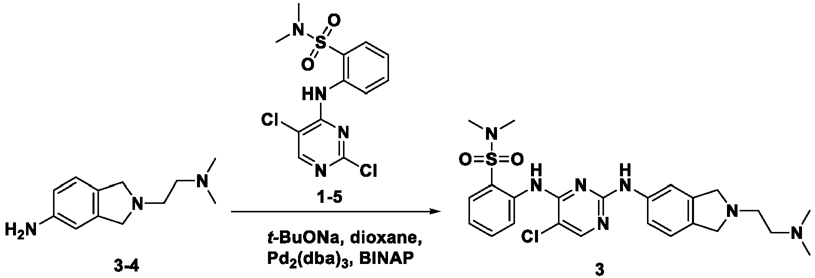

Compound 3-4 (100 mg, 0.49 mmol, 1.0 eq), compound 1-5 (140 mg, 0.41 mmol, 0.83 eq), t-BuONa (58.4 mg, 0.61 mmol, 1.25 eq), and BINAP (30.2 mg, 0.049 mmol, 0.1 eq) were dissolved in dioxane (3 mL), and Pd₂(dba)₃ (22.2 mg, 0.025 mmol, 0.05 eq) was added thereto. In a microwave reactor, the temperature was raised to 120°C, and the mixture reacted for 2 hours. The reaction solution was diluted with 10 mL of water and extracted with EtOAc for three times, 10 mL each time. The organic phases were combined, washed with 10 mL of saturated brine, dried over anhydrous sodium sulfate, filtered, and concentrated to obtain a crude product. The crude product was purified by preparative thin layer chromatography to obtain the target compound 3 (21.6 mg) as a white solid with a yield of 11.3%.

¹H NMR (300 MHz, DMSO-*d*₆): *δ* 9.50 (s, 1H), 9.27 (s, 1H), 8.49 (d, *J=* 7.8 Hz, 1H), 8.27 (s, 1H), 7.83 (dd, *J* = 7.8, 1.5 Hz, 1H), 7.73-7.67 (m, 1H), 7.53 (s, 1H), 7.41-7.31 (m, 2H), 7.08 (d, *J* = 8.1 Hz, 1H), 3.78 (d, *J* = 5.7 Hz, 4H), 2.80-2.72 (m, 2H), 2.64 (s, 6H), 2.47-2.40 (m, 2H), 2.21 (s, 6H).

LCMS: 516.2 ([M+H]⁺).

### Example 4

### Synthesis of 2-((5-chloro-2-((4-((3-(dimethylamino)pyrrolidin-1-yl)methyl)phenyl)amino)pyrimidin-4-yl)amino)-N,N-dimethylbenzenesulfonamide (Compound 4)

### Step 1: Synthesis of tert-butyl 3-(dimethylamino)pyrrolidine-1-carboxylate (4-2)

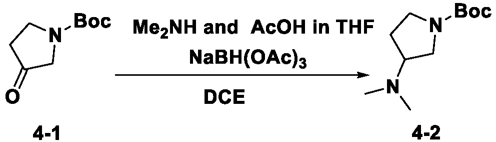

Tert-butyl-3-carbonylpyrrolidine-1-carboxylate (4-1, 2 g, 10.8 mol, 1.0 eq) was dissolved in 20 mL DCE, a solution of Me₂NH in THF (8 mL, 16.2 mmol, 1.5 eq) and AcOH (65 mg, 1.1 mmol, 0.1 eq) was added thereto, and the mixture reacted at room temperature for 15 minutes. NaBH(OAc)₃ (6.9 g, 32.4 mmol, 3.0 eq) was added to the reaction system, and the mixture reacted at room temperature for 16 hours. The reaction solution was diluted with 20 mL of water and extracted twice with DCM, 20 mL each time. The organic phases were combined, washed with 15 mL of saturated brine, dried over anhydrous sodium sulfate, filtered, and concentrated to obtain the crude product 4-2 (2.0 g), which was used directly in the next step.

¹H NMR (300 MHz, CDCl₃): δ 3.7-3.45 (m, 2H), 3.28-3.19 (dd, J = 9.6 Hz 1H), 3.08-2.98 (dd, J = 10.2 Hz, 1H), 2.72-2.60 (m, 1H), 2.22 (s, 6H), 2.10-1.90 (m, 1H), 1.80-1.60 (m, 1H), 1.41 (s, 9H).

### Step 2: Synthesis of N,N-dimethylpyrrolidin-3-amine hydrochloride (4-3)

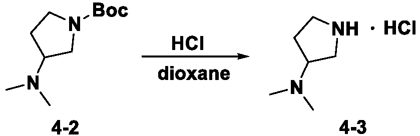

A hydrochloric acid solution of compound 4-2 (2.0 g, 9.3 mmol, 1.0 eq) was dissolved in 10 mL of dioxane, and the mixture reacted at room temperature for 2 hours. After the reaction was completed, the precipitate was filtered and collected to obtain the target compound 4-3 (1.3 g, hydrochloride) as a yellow solid with a yield in two-step reaction of 80.0%.

¹H NMR (300 MHz, DMSO-*d₆*): δ 11.68 (m, 1H), 4.02-3.90 (m, 1H), 3.56 (m, 1H), 3.45 (m, 2H), 3.20 (m, 1H), 2.78 (s, 6H), 2.33-2.18 (m, 2H).

### Step 3: Synthesis of N,N-dimethyl-1-(4-nitrophenylmethyl)pyrrolidin-3-amine (4-4)

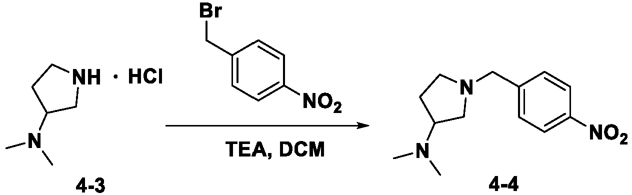

Compound 4-3 (1.1 g, 7.3 mmol, 1.0 eq) and 1-(bromomethyl)-4-nitrobenzene (1.65 g, 7.7 mmol, 1.05 eq) were dissolved in 10 mL of DCM, TEA (2.2 g, 21.9 mmol, 3.0 eq) was added thereto, and the mixture reacted at room temperature for 12 hours. The reaction solution was diluted with 10 mL of water and extracted twice with EtOAc, 20 mL each time. The organic phases were combined, washed with saturated brine for three times, 20 mL each time, dried over anhydrous sodium sulfate, filtered, and concentrated to obtain a crude product. The crude product was purified by silica gel column (DCM: MeOH = 20: 1) to obtain the target compound 4-4 (500 mg) as a yellow solid with a yield of 27.4%.

¹H NMR (300 MHz, CDCl₃): *δ* 8.21-8.17 (d, J = 8.4 Hz, 2H), 7.50-7.48 (d, J = 8.4 Hz,2H), 3.73 (s, 2H), 3.48-3.45 (m, 1H), 2.94-2.77 (m, 3H), 2.64 (m, 1H), 2.59 (s, 6H), 2.22-2.05 (m, 2H).

LCMS: 250.1 ([M+H]⁺).

### Step 4: Synthesis of 1-(4-aminophenylmethyl)-N,N-dimethylpyrrolidin-3-amine (4-5)

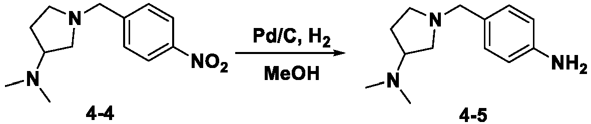

Compound 4-4 (400 mg, 1.6 mmol, 1.0 eq) was dissolved in 5 mL of MeOH, 80 mg Pd/C was added thereto, and the mixture reacted at room temperature under hydrogen for 2 hours. The reaction solution was filtered, and the filtrate was concentrated to obtain the target compound 4-5 (320 mg) as a white solid with a yield of 90.9%.

### Step 5: Synthesis of 2-((5-chloro-2-((4-((3-(dimethylamino)pyrrolidin-1-yl)methyl)phenyl)amino)pyrimidin-4-yl)amino)-N,N-dimethylbenzenesulfonamide (Compound 4)

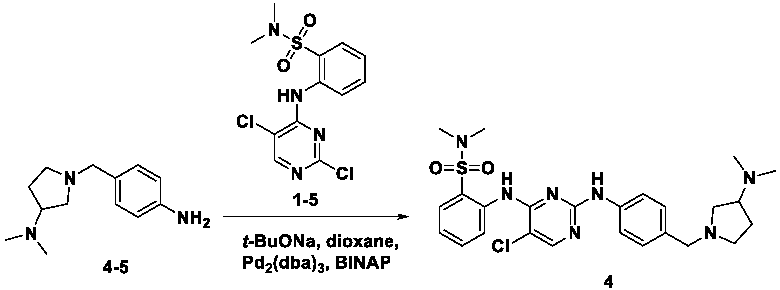

Compound 4-5 (170 mg, 0.78 mmol, 1.2 eq) and compound 1-5 (222 mg, 0.64 mmol, 1.0 eq) were dissolved in 5 mL of dioxane, and BINAP (48.3 mg, 0.08 mmol, 0.1 eq), t-BuONa (93.2 mg, 0.97 mmol, 1.5 eq), and Pd₂(dba)₃ (35.5 mg, 0.04 mmol, 0.05 eq) were added thereto. Under nitrogen protection, the temperature was raised to 100°C, and the mixture reacted for 12 hours. The reaction system was cooled to room temperature, quenched by adding 15 mL of water, and extracted with AcOH for three times, 25 mL each time. The organic phases were combined, washed twice with saturated brine (10 mL each time), dried over anhydrous sodium sulfate, filtered, and concentrated to obtain a crude product. The crude product was purified by preparative thin layer chromatography (DCM: MeOH = 10: 1) to obtain the target compound 4 (23.6 mg) as a yellow solid with a yield of 5.7%.

¹H NMR (400 MHz, DMSO-*d₆*): δ 9.51 (s, 1H), 9.32 (s, 1H), 8.60 (m, 1H), 8.28 (s, 1H), 7.83-7.80 (m, 1H), 7.69-7.67 (d, J = 7.2 Hz, 3H), 7.54-7.52 (d, J = 8.4 Hz, 2H), 7.38-7.34 (dd, J = 8.0 Hz, 1H), 7.16-7.14 (d, J = 8.4 Hz, 2H), 3.51-3.42 (m, 2H), 2.66 (m, 1H), 2.64 (m, 7H), 2.49 (m, 1H), 2.46-2.44 (m, 2H), 2.11 (s, 6H), 7.90-1.80 (m, 1H), 1.68-1.54 (m, 1H).

LCMS: 530.2 ([M+H]⁺).

### Example 5

### Synthesis of 2-((5-chloro-2-((4-((4-methylpiperazin-1-yl)methyl)phenyl)amino)pyrimidin-4-yl)amino)-N,N-dimethylpyridine-3-sulfonamide (Compound 5)

### Step 1: Synthesis of 2-chloro-N,N-dimethylpyridine-3-sulfonamide (5-2)

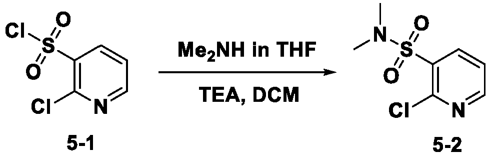

2-chloropyridine-3-sulfonyl chloride (5-1, 5.0 g, 24.3 mmol, 1.0 eq) was dissolved in 50 mL of DCM and TEA (4.9 g, 48.5 mmol, 2.0 eq). 2 mol/L of a solution of Me₂NH in THF (1.6 g, 36.4 mmol, 1.5 eq) was added thereto at room temperature. After the addition was complete, the mixture reacted at room temperature for 2 hours. The reaction mixture was diluted with 50 mL of water and extracted with DCM for three times, 50 mL each time. The organic phases were combined, washed with 50 mL of 1 mol/L hydrochloric acid, dried, and concentrated to obtain the target compound 5-2 (2.4 g, crude product) as an off-white oily liquid with a yield of 45.1%.

LCMS: 221.0 ([M+H]⁺).

### Step 2: Synthesis of 2-amino-N,N-dimethylpyridine-3-sulfonamide (5-3)

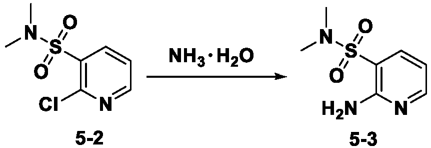

Compound 5-2 (2.4 g, 10.9 mmol) and 28 mL NH₃•H₂O were added to an autoclave, the temperature was raised to 150°C, and the mixture reacted for 12 hours. The reaction mixture was diluted with 150 mL of water and filtered. The filter residue was washed with 50 mL of water, and dried to obtain the target compound 5-3 (1.3 g) as a white solid with a yield of 59.3%.

LCMS: 202.1 ([M+H]⁺).

### Step 3: Synthesis of 2-((2,5-dichloropyrimidin-4-yl)amino)-N,N-dimethylpyridine-3-sulfonamide (5-4)

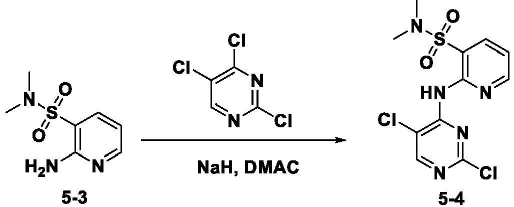

Compound 5-3 (1.0 g, 4.9 mmol, 1.0 eq) was dissolved in 10 mL of DMAC, and NaH (60% content in mineral oil, 792 mg, 19.8 mmol, 4.0 eq) was added thereto, and the mixture reacted at 25°C for 1 hour. Then, 2,4,5-trichloropyrimidine (3.17 g, 17.32 mmol, 3.5 eq) was slowly added into the system. After the addition was complete, the mixture reacted at 25°C for 3 hours. The reaction system was quenched by adding 10 mL of water, and extracted with EtOAc for three times, 15 mL each time. The organic phases were combined, washed with water for three times, 15 mL each time, and then washed with 15 mL of saturated brine, dried over anhydrous sodium sulfate, filtered, and concentrated under reduced pressure to obtain a crude product. The crude product was purified by silica gel column (DCM) to obtain the target compound 5-4 (800 mg) as a yellow solid with a yield of 46.9%.

¹H NMR (300 MHz, CDCl₃): *δ* 9.66 (s, 1H), 8.69 (dd, *J=* 4.8, 1.8 Hz, 1H), 8.38 (s, 1H), 8.17 (dd, *J* = 7.2, 1.8 Hz, 1H), 7.30-7.26 (m, 1H), 2.79 (s, 6H).

LCMS: 348.0 ([M+H]⁺).

### Step 4: Synthesis of 2-((5-chloro-2-((4-((4-methylpiperazin-1-yl)methyl)phenyl)amino)pyrimidin-4-yl)amino)-N,N-dimethylpyridine-3-sulfonamide (Compound 5)

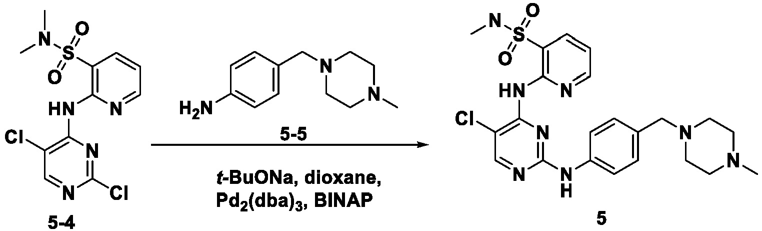

Compound 5-4 (100 mg, 0.28 mmol, 0.83 eq), 4-((4-methylpiperazin-1-yl)methyl)aniline (5-5, 71 mg, 0.34 mmol, 1.0 eq), BINAP (21.4 mg, 0.034 mmol, 0.1 eq), Pd₂(dba)₃ (15.6 mg, 0.017 mmol, 0.05 eq), and t-BuONa (41.2 mg, 0.43 mmol, 1.25 eq) were sequentially added to 3 mL of dioxane. The system was purged with nitrogen for 1 minute, the temperature was heated to 120°C, and the mixture reacted in a microwave reactor for 2 hours. The reaction solution was diluted with 10 mL of water and extracted with EtOAc for three times, 10 mL each time. The organic phases were combined, washed with 10 mL of saturated brine, dried, and concentrated to obtain a crude product. The crude product was purified by preparative liquid chromatography (acetonitrile/water, 5 mmol/L ammonium bicarbonate as an additive, 20 mL/min) to obtain the target compound 5 (19 mg) as a white solid with a yield of 12.8%.

¹H NMR (400 MHz, DMSO-*d*₆): *δ* 9.58 (s, 1H), 9.28 (brs, 1H), 8.82 (d, *J* = 3.2 Hz, 1H), 8.34 (s, 1H), 8.25 (dd, *J* = 8.0, 2.0 Hz, 1H), 7.73 (d, *J* = 8.4 Hz, 2H), 7.47 (dd, *J* = 8.0, 4.8 Hz, 1H), 7.10 (d, *J=* 8.4 Hz, 2H), 3.34 (s, 2H), 2.69 (s, 6H), 2.43-2.21 (m, 8H), 2.14 (s, 3H).

LCMS: 517.2 ([M+H]⁺).

### Example 6

### Synthesis of 4-((5-chloro-2-((4-((4-methylpiperazin-1-yl)methyl)phenyl)amino)pyrimidin-4-yl)amino)-N,N-dimethylpyridine-3-sulfonamide (Compound 6)

### Step 1: Synthesis of 4-chloro-N,N-dimethylpyridine-3-sulfonamide (6-2)

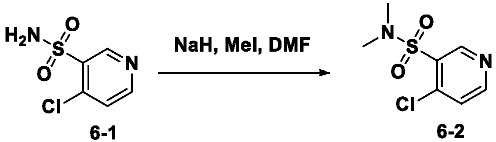

At 25°C, 4-chloropyridine-3-sulfonamide (6-1, 500 mg, 2.59 mmol, 1.0 eq) was dissolved in DMF, NaH (60% content in mineral oil, 228.8 mg, 5.72 mmol, 2.2 eq) was added to the solution, and the mixture reacted at room temperature for 1 hour. The temperature was lowered to 0°C, and MeI (811.9 mg, 5.72 mmol, 2.2 eq) was slowly added. Then, the temperature was raised to room temperature, and the system was stirred for 3 hours. When a completion of reaction was monitored by TLC, the reaction solution was quenched by adding 10 mL of water, extracted with DCM for three times, 15 mL each time, washed with 20 mL of saturated brine, and concentrated under reduced pressure to obtain a crude product. The crude product was purified by silica gel column (PE: EtOAc = 7: 3) to obtain the target compound 6-2 (600 mg) as an off-white oily liquid with a yield of 87.4%.

¹H NMR (300 MHz, DMSO-*d₆*): *δ* 8.34 (s, 1H), 8.14 (d, *J* = 6.0 Hz, 1H), 6.77 (d, *J* = 6.0 Hz, 1H), 2.67 (s, 6H).

### Step 2: Synthesis of 4-amino-N,N-dimethylpyridine-3-sulfonamide (6-3)

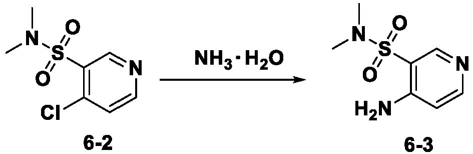

Compound 6-2 (3.0 g, 13.5 mmol) was dissolved in 60 mL NH₃•H₂O, and the mixture reacted in an autoclave at 120°C for 12 hours. The completion of the reaction was monitored by LC-MS. The reaction system was cooled to room temperature, quenched by adding 150 mL of water, filtered, washed with 50 mL of water, filtered, and dried to obtain the target compound 6-3 (1.4 g) as a white solid with a yield of 51.5%.

¹H NMR (300 MHz, DMSO-*d₆*): *δ* 8.33 (s, 1H), 8.13 (d, *J* = 6.0 Hz, 1H), 6.90 (brs, 2H), 6.77 (d, *J* = 6.0 Hz, 1H), 2.67 (s, 6H).

LCMS: 202.0 ([M+H]⁺).

### Step 3: Synthesis of 4-((2,5-dichloropyrimidin-4-yl)amino)-N,N-dimethylpyridine-3-sulfonamide (6-4)

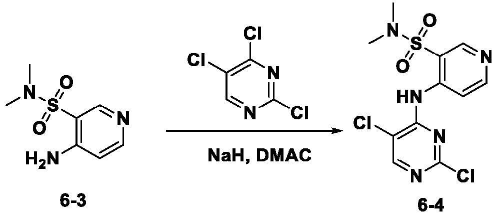

Compound 6-3 (800 mg, 3.96 mmol, 1.0 eq) was dissolved in 10 mL of DMAC, and NaH (60% content in mineral oil, 633.6 mg, 15.8 mmol, 4.0 eq) was added thereto. The mixture reacted at 25°C for 1 hour, and then compound 2,4,5-trichloropyrimidine (871.45 mg, 4.75 mmol, 1.2 eq) was slowly added. The temperature was controlled below 27°C during the addition. After the addition was completed, the mixture reacted at 25°C for 1 hour. The reaction solution was quenched by adding 10 mL of water, extracted with EtOAc for three times, 15 mL each time. The organic phases were combined, washed twice with saturated brine, 15 mL each time, and concentrated under reduced pressure to obtain a crude product. The crude product was purified by silica gel column (DCM) to obtain the target compound 6-4 (670 mg) as a yellow solid with a yield of 48.5%.

¹H NMR (300 MHz, DMSO-*d₆*): *δ* 10.06 (brs, 1H), 8.87-8.85 (m, 2H), 8.71 (s, 1H), 8.63 (s, 1H), 2.74 (s, 6H).

LCMS: 348.0 ([M+H]⁺).

### Step 4: Synthesis of 4-((5-chloro-2-((4-((4-methylpiperazin-1-yl)methyl)phenyl)amino)pyrimidin-4-yl)amino)-N,N-dimethylpyridine-3-sulfonamide

### (Compound 6)

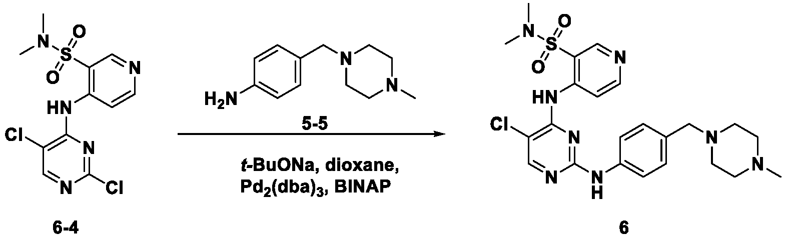

Compound 6-4 (150 mg, 0.43 mmol, 1.0 eq), compound 5-5 (95.1 mg, 0.46 mmol, 1.05 eq), BINAP (26.1 mg, 0.04 mmol, 0.1 eq), Pd₂(dba)₃ (19.2 mg, 0.02 mmol, 0.05 eq), and t-BuONa (50.4 mg, 0.54 mmol, 1.25 eq) were dissolved in 3 mL of dioxane. Under microwave condition, the mixture reacted at 120°C for 2 hours. The reaction system was cooled to room temperature, with 3 mL of water being added thereto, extracted with EtOAc for three times, 5 mL each time, and concentrated under reduced pressure to obtain a crude product. The crude product was separated and purified by preparative liquid chromatography (acetonitrile/water, 5 mmol/L ammonium bicarbonate as an additive, 20 mL/min) to obtain the target compound 6 (20.1 mg) as a white solid with a yield of 9.0%.

¹H NMR (400 MHz, DMSO-*d₆*): *δ* 9.64 (s, 1H), 8.84 (brs, 1H), 8.79 (m, 2H), 8.79 (s, 1H), 8.59 (d, *J* = 6.0 Hz, 1H), 8.35 (s, 1H), 7.56 (d, *J* = 8.0 Hz, 2H), 7.23 (d, *J* = 8.4 Hz, 2H), 3.41 (s, 2H), 2.75 (s, 6H), 2.43-2.22 (m, 8H), 2.14 (s, 3H).

LCMS: 517.2 ([M+H]⁺).

### Example 7

### Synthesis of 3-((5-chloro-2-((4-((4-methylpiperazin-1-yl)methyl)phenyl)amino)pyrimidin-4-yl)amino)-N,N-dimethylpyridine-2-sulfonamide (Compound 7)

### Step 1: Synthesis of 2-(phenylmethylthio)-3-nitropyridine (7-2)

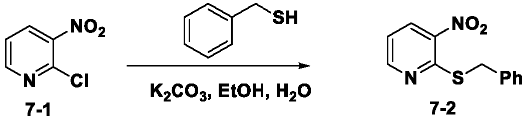

2-chloro-3-nitropyridine (7-1, 5.0 g, 32 mmol, 1.0 eq) and phenylmethyl mercaptan (4.3 g, 35.2 mmol, 1.1 eq) were dissolved in a mixed solution of 150 mLEtOH and 30 mL H₂O, K₂CO₃ was added thereto, and the mixture reacted at room temperature for 12 hours. The reaction solution was quenched by adding 100 mL of water, filtered, and then washed with 30 mL of water to obtain a yellow crude product. The crude product was dried in vacuum to obtain the target compound 7-2 (8.3 g) as a yellow solid with a yield of 95.8%.

¹H NMR (300 MHz, DMSO-*d₆*): *δ* 8.88 (dd, *J* = 4.8, 1.8 Hz, 1H), 8.63 (dd, *J* = 8.4, 1.8 Hz, 1H), 7.50-7.48 (m, 1H), 7.47-7.43 (m, 2H), 7.36-7.26 (m, 3H), 4.50 (s, 2H).

### Step 2: Synthesis of N,N-dimethyl-3-nitropyridine-2-sulfonamide (7-3)

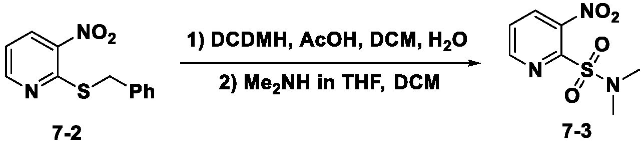

Compound 7-2 (1.0 g, 4.1 mmol, 1.0 eq) was dissolved in a mixed solution of AcOH (2 mL), H₂O (4 mL), and DCM (24 mL), and the system was cooled to 0°C. DCDMH (2.4 g, 12.2 mmol, 3.0 eq) was added thereto, the temperature was raised to 25°C, and the mixture reacted for 4 hours. The completion of the reaction was monitored by TLC and LC-MS. The mixture was washed sequentially with 16 mL of 5% sodium dithionite solution and 16 mL of 10% potassium dihydrogen phosphate solution and concentrated to obtain a residue. The residue was diluted with 50 mL of DCM, and cooled to 0°C. A solution of Me₂NH in THF (2 mmol/L, 6 mL) was slowly added. After the addition was complete, the mixture reacted at 25°C for 20 minutes. The reaction solution was quenched with 5 mL of saturated citric acid solution. The organic phase was concentrated to obtain the target compound 7-3 (840 mg) as a yellow solid with a yield of 88.5%.

¹H NMR (300 MHz, DMSO-*d₆*): *δ* 8.96 (dd, *J* = 4.5, 1.5 Hz, 1H), 8.59 (dd, *J=* 8.1, 1.5 Hz, 1H), 7.98 (dd, *J=* 8.1 Hz, 4.5 Hz, 1H), 2.97 (s, 6H).

LCMS: 232.0 ([M+H]⁺).

### Step 3: Synthesis of 3-amino-N,N-dimethylpyridine-2-sulfonamide (7-4)

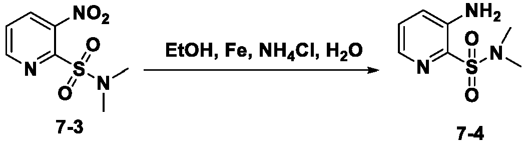

Compound 7-3 (840 mg, 3.63 mmol, 1.0 eq) and Fe (460 mg, 36.3 mmol, 10.0 eq) were dissolved in 8 mLEtOH, and an aqueous solution of NH₄Cl (230 mg, 18.15 mmol, 5.0 eq) was added thereto at room temperature. The temperature was raised to 105°C, and the mixture reacted for 1.5 hours. The reaction solution was concentrated under reduced pressure, then diluted with 10 mL of water, extracted with EtOAc for three times, 50 mL each time, dried over anhydrous sodium sulfate, filtered, and concentrated under reduced pressure to obtain the target compound 7-4 (640 mg) as a brown solid with a yield of 87.7%.

¹H NMR (300 MHz, DMSO-*d₆*): *δ* 7.95 (s, 1H), 7.84 (dd, *J* = 3.6, 1.8 Hz, 1H), 7.31-7.29 (m, 1H), 6.10 (brs, 2H), 2.83 (s, 6H).

LCMS: 202.0 ([M+H]⁺).

### Step 4: Synthesis of 3-((2,5-dichloropyrimidin-4-yl)amino)-N,N-dimethylpyridine-2-sulfonamide (7-5)

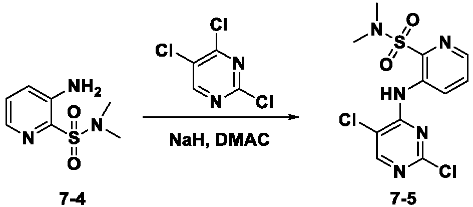

NaH (in mineral oil with a concentration of 60%, 320 mg, 8 mmol, 4.0 eq) was dissolved in 8 mL of DMAC, compound 7-4 (400 mg, 1.98 mmol, 1.0 eq) was added thereto at 25°C, and the mixture reacted at room temperature for 1 hour. The temperature was controlled to be lower than 27°C. Compound 2,4,5-trichloropyrimidine (435 mg, 2.4 mmol, 1.2 eq) was slowly added, and the mixture reacted at 25°C for 1 hour. The reaction solution was concentrated in vacuum to obtain a crude product. The crude product was purified by silica gel column (PE: EtOAc = 3: 1) to obtain the target compound 7-5 (320 mg) as a white solid with a yield of 46.4%.

¹H NMR (300 MHz, DMSO-*d₆*): *δ* 9.80 (brs, 1H), 8.71 (dd, *J* = 8.4, 1.5 Hz, 1H), 8.57 (s, 1H), 8.53 (dd, *J* = 4.5, 1.5 Hz, 1H), 7.81 (dd, *J* = 8.4, 1.5 Hz, 1H), 2.94 (s, 3H), 2.93 (s, 3H).

LCMS: 348.0 ([M+H]⁺).

### Step 5: Synthesis of 3-((5-chloro-2-((4-((4-methylpiperazin-1-yl)methyl)phenyl)amino)pyrimidin-4-yl)amino)-N,N-dimethylpyridine-2-sulfonamide (Compound 7)

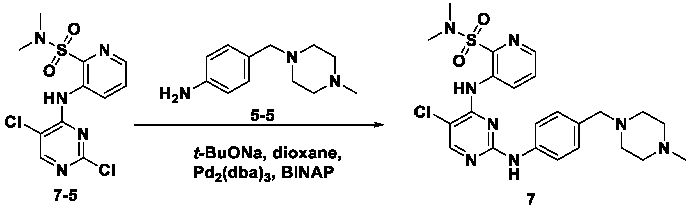

Compound 7-5 (150 mg, 4.3 mmol, 1.0 eq), compound 5-5 (95 mg, 4.6 mmol, 1.05 eq), *t*-BuONa (100 mg, 5.4 mmol, 1.25 eq), and BINAP (26.1 mg, 0.43 mmol, 0.1 eq) were dissolved in 3 mL of dioxane. Under nitrogen protection, Pd₂(dba)₃ (19.2 mg, 0.21 mmol, 0.05 eq) was added at room temperature. Under microwave condition, the mixture reacted at 120°C for 2 hours. The reaction system was cooled to room temperature, quenched by adding 3 mL of water, extracted with EtOAc for three times, 5 mL each time, and concentrated in vacuum to obtain a crude product. The crude product was separated and purified by preparative liquid chromatography (acetonitrile/water, 5 mmol/L ammonium bicarbonate as an additive, 20 mL/min) to obtain the target compound 7 (11.7 mg) as a white solid with a yield of 5.3%.

¹H NMR (400 MHz, CD₃OD): *δ* 9.23 (d, *J* = 8.4 Hz, 1H), 8.35 (dd, *J* = 4.4 Hz, 1.2 Hz, 1H), 8.19 (s, 1H), 7.58-7.54 (m, 3H), 7.27 (d, *J=* 8.8 Hz, 2H), 3.61 (s, 2H), 3.01 (s, 6H), 2.99-2.54 (m, 8H), 2.52 (s, 3H).

¹H NMR (400 MHz, DMSO-*d₆*): *δ* 9.58 (s, 1H), 9.43 (s, 1H), 9.10 (brs, 1H), 8.43 (dd, *J* = 4.4, 1.2 Hz, 1H), 8.13 (s, 1H), 7.66 (dd, *J* = 8.4 Hz, 4.4 Hz, 1H), 7.53 (d, *J* = 7.6 Hz, 2H), 7.18 (d, *J=* 8.0 Hz, 2H), 3.45 (s, 2H), 2.93 (s, 7H), 2.68-2.51 (m, 4H), 2.44-2.22 (m, 6H).

LCMS: 517.2 ([M+H]⁺).

### Example 8

### Synthesis of 2-((5-chloro-2-((4-((3-methyl-3,8-diazabicyclo[3.2.1]octan-8-yl)methyl)phenyl)amino)pyrimidin-4-yl)amino)-N,N-dimethylbenzenesulfonamide (Compound 8)

### Step 1: Synthesis of 2-((5-chloro-2-((4-(hydroxymethyl)phenyl)amino)pyrimidin-4-yl)amino)-N,N-dimethylbenzenesulfonamide (8-1)

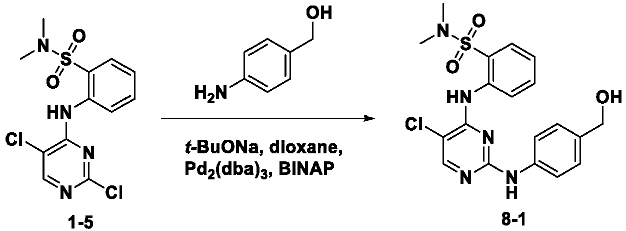

Compound 1-5 (1.0 g, 2.89 mmol, 1.0 eq) was dissolved in 10 mL of dioxane, and 4-aminophenylmethanol (430 mg, 3.48 mmol, 1.2 eq), Pd₂(dba)₃ (159 mg, 0.17 mmol, 0.05 eq), BINAP (217 mg, 0.35 mmol, 0.1 eq), and *t*-BuONa (418 mg, 4.35 mmol, 1.5 eq) were added thereto. Under nitrogen protection, the temperature was raised to 100°C, and the mixture reacted for 12 hours. The reaction system was cooled to room temperature and added with 15 mL of water thereto, and extracted for three times with EtOAc, 15 mL each time. The organic phases were combined, washed twice with saturated brine, 15 mL each time, then dried over anhydrous sodium sulfate, filtered, and concentrated to obtain a crude product. The crude product was purified by silica gel column (PE: EtOAc = 1: 3) to obtain the target compound 8-1 (600 mg) as a yellow solid with a yield of 47.9%.

LCMS: 434.1 ([M+H]⁺).

### Step 2: Synthesis of 4-((5-chloro-4-((2-(N,N-dimethylaminosulfonyl)phenyl)amino)pyrimidin-2-yl)amino) phenylmethyl methanesulfonate (8-2)

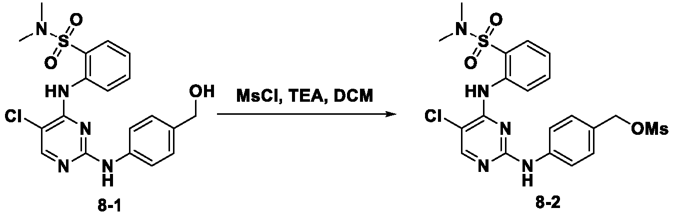

Compound 8-1 (600 mg, 1.38 mmol, 1.0 eq) was dissolved in 10 mL of DCM, and TEA (279 mg, 2.76 mmol, 2.0 eq) was added thereto at room temperature. The mixed system was cooled to 0°C, MsCl (236 mg, 2.07 mmol, 1.5 eq) was added thereto, and the mixture was heated to room temperature and reacted for 4 hours. 20 mL of water was added to the reaction solution, and then the reaction solution was extracted with DCM for three times, 20 mL each time. The organic phases were combined, washed twice with saturated brine (20 mL each time), dried over anhydrous sodium sulfate, and concentrated to obtain the crude product 8-2 (750 mg) as a yellow solid, which was used directly in the next step of reaction.

### Step 3: Synthesis of 2-((5-chloro-2-((4-((3-methyl-3,8-diazabicyclo[3.2.1]octan-8-yl)methyl)phenyl)amino)pyrimidin-4-yl)amino)-N,N-dimethylbenzenesulfonamide (Compound 8)

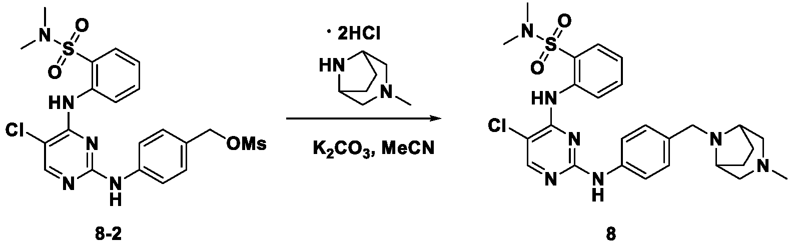

Compound 8-2 (250 mg, 0.49 mmol, 1.0 eq) and 3-methyl-3,8-diazabicyclo[3.2.1]octane hydrochloride (8-3, 107 mg, 0.54 mmol, 1.1 eq) were dissolved in 5 mL of MeCN, and K₂CO₃ (340 mg, 2.45 mmol, 5.0 eq) was added thereto. Under nitrogen protection, the temperature was raised to 80°C, and the mixture reacted for 4 hours. The reaction system was cooled to room temperature and added with 15 mL of water, and extracted with EtOAc for three times, 20 mL each time. The organic phases were combined, washed twice with saturated brine (20 mL each time), dried over anhydrous sodium sulfate, filtered, and concentrated to obtain a crude product. The crude product was purified by preparative liquid chromatography (acetonitrile/water, 5 mmol/L ammonium bicarbonate as an additive, 20 mL/min) to obtain the target compound 8 (19 mg) as a white solid with a yield of 7.2%.

¹H NMR (400 MHz, DMSO-*d*₆): *δ* 9.50 (s, 1H), 9.32 (brs, 1H), 8.58-8.55 (m, 1H), 8.28 (s, 1H), 7.83 (d, *J=* 1.4 Hz, 1H), 7.68 (t, *J=* 7.2 Hz, 1H), 7.52 (d, *J=* 8.4 Hz, 2H), 7.36 (t, *J=* 7.2 Hz, 1H), 7.14 (d, *J=* 8.4 Hz, 2H), 3.36 (s, 2H), 2.97 (s, 2H), 2.64 (s, 6H), 2.48-2.45 (m, 2H), 2.19-2.16 (m, 2H), 2.13 (s, 3H), 1.85-1.82 (m, 2H), 1.69-1.68 (m, 2H).

LCMS: 542.2 ([M+H]⁺).

### Example 9

### Synthesis of 2-((5-chloro-2-((4-((8-methyl-3,8-diazabicyclo[3.2.1]octan-3-yl)methyl)phenyl)amino)pyrimidin-4-yl)amino)-N,N-dimethylbenzenesulfonamide (Compound 9)

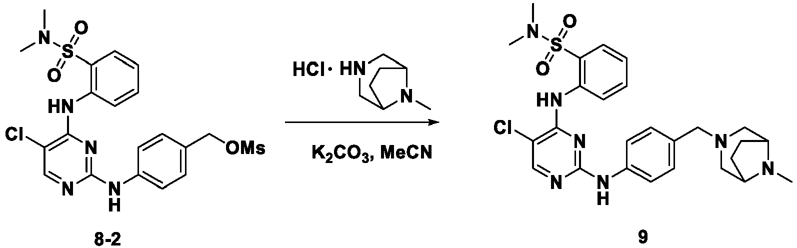

Compound 8-2 (250 mg, 0.49 mmol) and 8-methyl-3,8-diazabicyclo[3.2.1]octane hydrochloride (107 mg, 0.54 mmol) were dissolved in 5 mL of MeCN, and K₂CO₃ (340 mg, 2.45 mmol) was added to the system. Under nitrogen protection, the temperature was raised to 80°C, and the mixture reacted for 2 hours. The completion of the reaction was monitored by TLC. Then, the reaction system was cooled to room temperature and added with 15 mL of water, and extracted with EtOAc for three times, 20 mL each time. The organic phases were combined, washed twice with saturated brine, 20 mL each time, then dried over anhydrous sodium sulfate, filtered, and concentrated to obtain a crude product. The crude product was separated and purified by preparative liquid chromatography (acetonitrile/water, 5 mmol/L ammonium bicarbonate as an additive, 20 mL/min) to obtain the target compound 9 (34.2 mg) as a white solid with a yield of 12.9%.

¹H NMR (400 MHz, DMSO-*d₆*): *δ* 9.50 (s, 1H), 9.32 (brs, 1H), 8.58-8.55 (m, 1H), 8.28 (s, 1H), 7.83 (d, *J=* 1.4 Hz, 1H), 7.68 (t, *J=* 7.2 Hz, 1H), 7.52 (d, *J=* 8.4 Hz, 2H), 7.36 (t, *J=* 7.2 Hz, 1H), 7.14 (d, *J=* 8.4 Hz, 2H), 3.36 (s, 2H), 2.97 (s, 2H), 2.64 (s, 6H), 2.48-2.45 (m, 2H), 2.19-2.16 (m, 2H), 2.13 (s, 3H), 1.85-1.82 (m, 2H), 1.69-1.68 (m, 2H).

LCMS: 542.2 ([M+H]⁺).

### Example 10

### Synthesis of 2-((5-chloro-2-((4-(((3R,5S)-3,4,5-trimethylpiperazin-1-yl)methyl)phenyl)amino)pyrimidin-4-yl)amino)-N,N-dimethylbenzenesulfonamide (Compound 10)

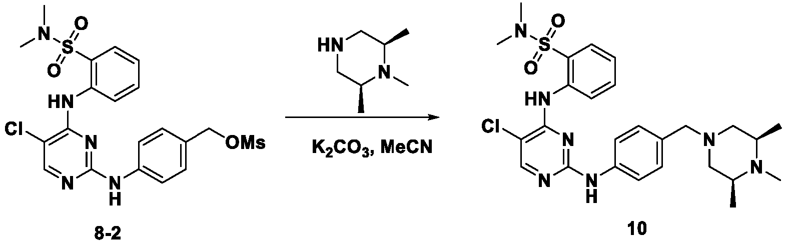

Compound 8-2 (250 mg, 0.49 mmol) and (2R, 6S)-1,2,6-trimethylpiperazine (69.3 mg, 0.54 mmol) were dissolved in 5 mL of MeCN, and K₂CO₃ (340 mg, 2.45 mmol) was added to the system. Under nitrogen protection, the mixture was heated to 80°C and reacted for 2 hours. The completion of the reaction was showed by LC-MS monitoring. Then, the reaction system was cooled to room temperature and added with 15 mL of water, and extracted with EtOAc for three times, 20 mL each time. The organic phases were combined, washed with saturated brine twice, 20 mL each time, and then dried over anhydrous sodium sulfate, filtered, and concentrated to obtain a crude product. The crude product was separated and purified by preparative liquid chromatography (acetonitrile/water, 5 mmol/L ammonium bicarbonate as an additive, 20 mL/min) to obtain the target compound 10 (20.8 mg) as a white solid with a yield of 7.8%.

¹H NMR (400 MHz, DMSO-*d₆*): *δ* 9.52 (s, 1H), 9.33 (s, 1H), 8.59 (d, *J =* 9.2 Hz, 1H), 8.28 (s, 1H), 7.82 (dd, *J=* 8.0, 1.6 Hz, 1H), 7.69 (t, *J=* 8.0 Hz, 1H), 7.53 (d, *J=* 8.4 Hz, 2H), 7.36 (t, *J* = 7.2 Hz, 1H), 7.15 (d, *J* = 8.4 Hz, 2H), 3.29-3.27 (m, 2H), 2.70-2.57 (m, 9H), 2.17-2.08 (m, 4H), 1.73 (t, *J=* 10.0 Hz, 2H), 0.93 (d, *J=* 6.0 Hz, 6H).

LCMS: 544.1 ([M+H]⁺).

### Example 11

### Synthesis of 2-((5-chloro-2-((2-(2-methoxyethyl)-1,2,3,4-tetrahydroisoquinolin-6-yl)amino)pyrimidin-4-yl)amino)-N,N-dimethylbenzenesulfonamide formate (Compound 11)

### Step 1: Synthesis of 2-(3-nitrophenyl)acetamide (11-1)

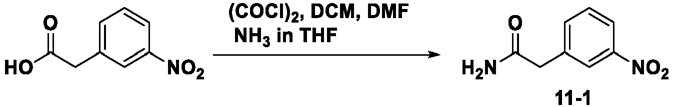

2-(3-nitrophenyl)acetic acid (10.0 g, 55.2 mmol, 1.0 eq) and 3 drops of DMF were dissolved in 100 mL of DCM, (COCl)₂ (7.0 g, 55.2 mmol, 1.0 eq) was added dropwise thereto, and the mixture reacted at room temperature for 2 hours. A solution of NH₃ in THF (500 mL) was added thereto, and the mixture reacted for 1 hour. The reaction mixture was extracted twice with DCM, 500 mL each time. The organic phases were combined, washed with 500 mL of saturated brine, dried over anhydrous sodium sulfate, filtered, and concentrated to obtain a crude product. The crude product was purified by silica gel column (DCM: MeOH = 10: 1) to obtain the target compound 11-1 (6 g) as a white solid with a yield of 60.3%.

¹H NMR (300 MHz, DMSO-*d*₆): *δ* 8.15 (s, 1H), 8.13-8.09 (m, 1H), 7.72 (d, *J* = 7.8 Hz, 1H), 7.64-7.58 (m, 2H), 7.02 (brs, 1H), 3.57 (s, 2H).

LCMS: 181.1 ([M+H]⁺).

### Step 2: Synthesis of 2-(3-nitrophenyl)ethane-1-amine hydrochloride (11-2)

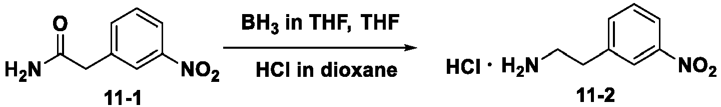

A solution of BH₃ in THF (60 mL) under stirring was cooled to 0°C, and compound 11-1 (6.0 g, 33.3 mmol, 1.0 eq) in THF solution was added thereto. The mixture was heated to room temperature and reacted for 2 hours. The mixture was then heated to 70°C and reacted for 3 hours. The completion of the reaction was indicated by liquid chromatogram (LC) monitoring. The reaction system was cooled to room temperature and carefully quenched with 50 mL of 3N hydrochloric acid. The reaction system was cooled to room temperature, dissolved with 60 mL of 3M sodium hydroxide solution, and then extracted with DCM twice, 100 mL each time. The organic phases were combined, dried over anhydrous sodium sulfate, filtered, and concentrated to obtain a crude product. The crude product was dissolved in 50 mL of DCM, 10 mL of 4N HCl in dioxane solution was added thereto, and the mixture reacted for 1 hour. The reaction system was filtered and dried to obtain the target compound 11-2 (4.8 g) as an off-white solid with a yield of 86.8%.

¹H NMR (300 MHz, DMSO-*d*₆): *δ* 8.19-8.12 (m, 2H), 8.09 (brs, 2H), 7.77 (d, *J=* 7.5 Hz, 1H), 7.65 (t, *J=* 7.5 Hz, 1H), 3.21-3.01 (m, 4H).

LCMS: 167.1 ([M-HCl+H]⁺).

### Step 3: Synthesis of N-(3-nitrophenylethyl)methanesulfonamide (11-3)

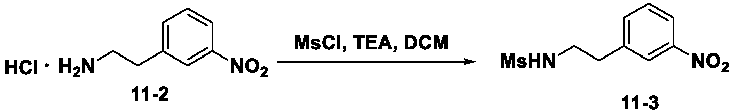

At room temperature, compound 11-2 (4.4 g, 21.7 mmol, 1.0 eq) was dissolved in 40 mL of DCM, and TEA (6.4 g, 63 mmol, 3.0 eq) was added thereto. The temperature was lowered to -20°C, a solution of MsCl (3.0 g, 26 mmol, 1.2 eq) in DCM (20 mL) was slowly added, and the mixture reacted at -20°C for 20 minutes. Then, the temperature was raised to room temperature and the reaction was carried out for 2 hours. After the reaction was completed, 25 mL of water was added to the system, and the system was extracted with DCM for three times, 50 mL each time. The organic phases were combined, washed with 100 mL of saturated brine, dried over anhydrous sodium sulfate, filtered, and concentrated to obtain the target compound 11-3 (3.4 g) as a white solid with a yield of 94.3%.

¹H NMR (300 MHz, DMSO-*d*₆): *δ* 8.16 (s, 1H), 8.15-8.08 (m, 1H), 7.75 (d, *J=* 7.8 Hz, 1H), 7.62 (t, *J* = 7.8 Hz, 1H), 7.12 (t, *J* = 5.7 Hz, 1H), 3.29-3.22 (m, 2H), 2.92 (t, *J* = 7.2 Hz, 2H), 2.86 (s, 3H).

LCMS: 267.0 ([M+Na]⁺).

### Step 4: Synthesis of 6-nitro-1,2,3,4-tetrahydroisoquinoline (11-4)

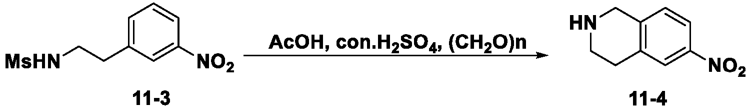

At 0°C, 50 mL of concentrated H₂SO₄ was added to 35 mL of AcOH, then a solution of compound 11-3 (5.4 g, 22.4 mmol, 1.0 eq) in AcOH (25 mL) was added, and the mixture was stirred until the reaction was completed. Into the mixture, (CH₂O)ₙ (637 mg, 22.4 mmol, 1.0 eq) was added, and the mixture was cooled to 0°C and reacted for 30 minutes. Then, the mixture was heated to 45°C and reacted for 12 hours. After the completion of the reaction was monitored by LC-MS, the reaction system was cooled to room temperature, quenched with 50 mL of ice water, and extracted twice with EtOAc, 100 mL each time. The organic phases were combined, washed twice with saturated brine, 50 mL each time, dried over anhydrous sodium sulfate, filtered, and concentrated to obtain the crude compound 11-4 (2.9 g) as a yellow solid, which was used directly in the next step.

LCMS: 179.1 ([M+H]⁺).

### Step 5: Synthesis of tert-butyl-6-nitro-3,4-dihydroisoquinoline-2(1H)-carboxylate (11-5)

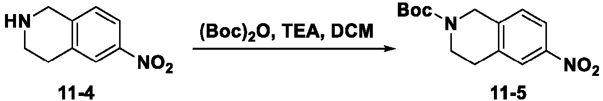

Compound 11-4 (2.9 g, 16.3 mmol, 1.0 eq) was dissolved in 25 mL of DCM, TEA (3.3 g, 32.6 mmol, 2.0 eq) and (Boc)₂O (4.0 g, 18.0 mmol, 1.1 eq) were added thereto, and the mixture reacted at room temperature for 4 hours. After the reaction was completed, 25 mL of water was added thereto, and the mixture was extracted with DCM for three times, 25 mL each time. The organic phases were combined, washed with saturated brine twice, 20 mL each time, dried over anhydrous sodium sulfate, filtered, and concentrated to obtain a crude product. The crude product was purified by silica gel column (PE: EtOAc = 5: 1) to obtain the target compound 11-5 (1.5 g) as a yellow solid with a yield of 33.1%.

¹H NMR (300 MHz, DMSO-*d*₆): *δ* 8.08 (d, *J=* 2.1 Hz, 1H), 8.04 (dd, *J=* 8.4, 2.4 Hz, 1H), 7.49 (d, *J* = 8.4 Hz, 1H), 4.63 (s, 2H), 3.59 (t, *J* = 6.0 Hz, 2H), 2.92 (t, *J* = 6.0 Hz, 2H), 1.44 (s, 9H).

LCMS: 223.0 ([M-56+H]⁺).

### Step 6: Synthesis of tert-butyl-6-amino-3,4-dihydroisoquinoline-2(1H)-carboxylate (11-6)

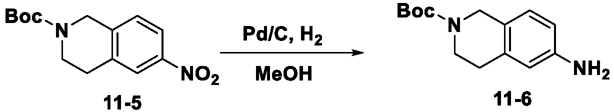

Compound 11-5 (2 g, 7.2 mol, 1.0 eq) was dissolved in 20 mL of MeOH, and 200 mg Pd/C was added thereto at room temperature. Under hydrogen protection, the mixture reacted at room temperature for 4 hours. The completion of the reaction was monitored by LC-MS. The mixture was filtered, and the filtrate was concentrated to obtain the target compound 11-6 (1.8 g) as a yellow solid with a yield of 92.5%.

LCMS: 249.1 ([M+H]⁺).

### Step 7: Synthesis of tert-butyl 6-((5-chloro-4-((2-(N,N-dimethylaminosulfonyl)phenyl)amino)pyrimidin-2-yl)amino)-3,4-dihydroisoquinoline-2(1H)-carboxylate (11-7)

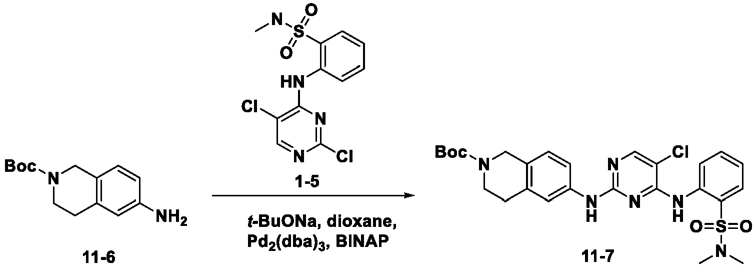

Compound 11-6 (500 mg, 2.0 mmol, 1.2 eq) and compound 1-5 (580 mg, 1.7 mmol, 1.0 eq) were dissolved in 10 mL of dioxane, and BINAP (124 mg, 0.2 mmol, 0.1 eq), t-BuONa (240 mg, 2.5 mmol, 1.5 eq), and Pd₂(dba)₃ (92 mg, 0.1 mmol, 0.05 eq) were added thereto. Under nitrogen protection, the temperature was raised to 100°C, and the mixture reacted for 12 hours. After the reaction was completed, the mixture was cooled to room temperature, quenched with 15 mL of water, and extracted with EtOAc for three times, 25 mL each time. The organic phases were combined, washed twice with saturated brine (10 mL each time), dried over anhydrous sodium sulfate, filtered, and concentrated to obtain a crude product. The crude product was purified by silica gel column (PE: EtOAc = 2: 1) to obtain the target compound 11-7 (350 mg) as a brown solid with a yield of 31.4%.

¹H NMR (300 MHz, DMSO-*d*₆): *δ* 9.51 (s, 1H), 9.28 (brs, 1H), 8.52 (d, *J=* 7.8 Hz, 1H), 8.29 (s, 1H), 7.84 (dd, *J* = 8.1, 1.5 Hz, 1H), 7.75-7.69 (m, 1H), 7.47-7.43 (m, 1H), 7.42-7.35 (m, 2H), 7.04 (d, *J=* 8.7 Hz, 1H), 4.43 (s, 2H), 3.53 (t, *J=* 6.0 Hz, 2H), 2.68-2.61 (m, 8H), 1.44 (s, 9H).

LCMS: 559.2 ([M+H]⁺).

### Step 8: Synthesis of 2-((5-chloro-2-((1,2,3,4-tetrahydroisoquinolin-6-yl)amino)pyrimidin-4-yl)amino hydrochloride (11-8)

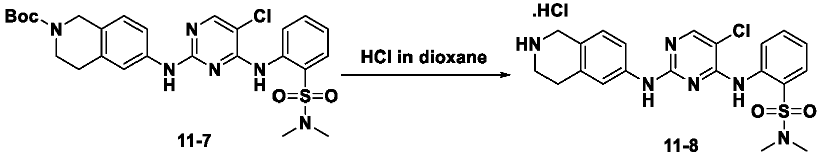

Compound 11-7 (350 mg, 0.6 mmol) was added to a solution of HCl in dioxane (6 mL), and the mixture reacted at room temperature for 12 hours. After the reaction was completed, the target compound 11-8 (250 mg) was obtained by filtration as a yellow solid with a yield of 91.0%.

¹H NMR (300 MHz, DMSO-*d*₆): *δ* 9.87 (s, 1H), 9.49 (brs, 3H), 8.43 (d, *J =* 7.8 Hz, 1H), 8.35 (s, 1H), 7.86 (dd, *J* = 7.8, 1.2 Hz, 1H), 7.80-7.73 (m, 1H), 7.47-7.37 (m, 3H), 7.01 (d, *J* = 8.4 Hz, 1H), 4.17 (brs, 2H), 3.38-3.26 (m, 2H), 2.86 (t, *J* = 6.0 Hz, 1H, 2H), 2.56 (s, 6H).

LCMS: 459.1 ([M-HCl+H]⁺).

### Step 9: Synthesis of 2-methoxyacetaldehyde (11-9)

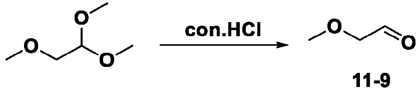

1,1,2-trimethoxyethane (1 g, 6.2 mol) was dissolved in concentrated HCl (5 mL), and the mixture was heated to 40°C and reacted for 3 hours. The completion of the reaction was monitored by TLC. Then, the reaction system was extracted with 5 mL of DCM to obtain compound 11-9 in DCM solution, which was directly used in the next step of reaction.

### Step 10: Synthesis of 2-((5-chloro-2-((2-(2-methoxyethyl)-1,2,3,4-tetrahydroisoquinolin-6-yl)amino)pyrimidin-4-yl)amino)-N,N-dimethylbenzenesulfonamide formate (11)

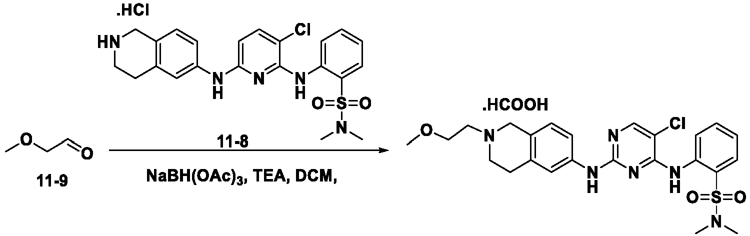

Compound 11-9 was dissolved in 4 mL of DCM, and compound 11-8 (11-2, 150 mg, 0.3 mmol, 1.0 eq) and TEA (90.9 mg, 0.9 mmol, 3.0 eq) were added at room temperature. After the addition was complete, the mixture reacted for 10 minutes. Then, NaBH(OAc)₃ (125 mg, 0.6 mmol, 2.0 eq) was added to the system, and the mixture reacted at room temperature for 30 minutes. The completion of the reaction was monitored by LC-MS. 10 mL of water was added to the reaction system, and then the reaction system was extracted with EtOAc for three times, 15 mL each time. The organic phases were combined, washed twice with saturated brine (10 mL each time), then dried over anhydrous sodium sulfate, filtered, and concentrated to obtain a crude product. The crude product was separated and purified by preparative liquid chromatography (acetonitrile/water, 0.1% formic acid as an additive, 20 mL/min) to obtain a formate of the target compound 11 (60.8 mg, formate) as a yellow solid with a yield of 38.7%.

¹H NMR (400 MHz, DMSO-*d*₆): *δ* 9.46 (s, 1H), 9.27 (s, 1H), 8.58-8.49 (m, 1H), 8.28 (s, 1H), 8.14 (s, 2H), 7.82 (dd, *J* = 8.0, 1.6 Hz, 1H), 7.71 (t, *J* = 7.2 Hz, 1H), 7.39-7.31 (m, 3H), 6.93 (d, *J* = 8.4 Hz, 1H), 3.66 (s, 2H), 3.55 (t, *J* = 5.6 Hz, 2H), 3.28 (s, 3H), 2.80-2.66 (m, 6H), 2.64 (s, 6H).

LCMS: 517.2 ([M-HCOOH+H]⁺).

### Example 12

### Synthesis of 2-((5-chloro-2-((2-(2-(methylamino)ethyl)-1,2,3,4-tetrahydroisoquinolin-6-yl)amino)pyrimidin-4-yl)amino)-N,N-dimethylbenzenesulfonamide (Compound 12)

### Step 1: Synthesis of tert-butyl-(2-(6-((5-chloro-4-((2-(N,N-dimethylaminosulfonyl)phenyl)amino)pyrimidin-2-yl)amino)-3,4-dihydroisoquinolin-2(1H)-yl)ethyl)(methyl)carbamate (Compound 12-1)

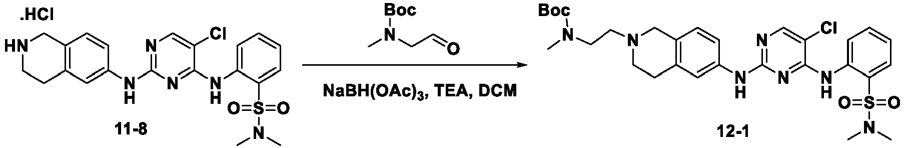

Compound tert-butyl-methyl (2-carbonylethyl) carbamate (113 mg, 0. 65mmol, 1.5eq) was dissolved in SmL of DCM, compound 11-8 (200mg, 0. 44mmol, 1.0eq) and TEA (45mg, 0.44mmol) were added thereto, and the mixture reacted for 10 minutes. Then, NaBH(OAc)₃ (168 mg, 0.79 mmol, 1.8 eq) was added, and the mixture reacted for 30 minutes. After the reaction was completed, 10 mL of water was added to the reaction solution, and the reaction solution was extracted with EtOAc for three times, 15 mL each time. The organic phases were combined, washed twice with saturated brine (10 mL each time), dried over anhydrous sodium sulfate, filtered, and concentrated to obtain the target compound 12-1 (350 mg) as a white solid with a yield of 87.5%.

LCMS: 616.2 ([M+H]⁺).

### Step 2: Synthesis of 2-((5-chloro-2-((2-(2-(methylamino)ethyl)-1,2,3,4-tetrahydroisoquinolin-6-yl)amino)pyrimidin-4-yl)amino)-N,N-dimethylbenzenesulfonamide (Compound 12)

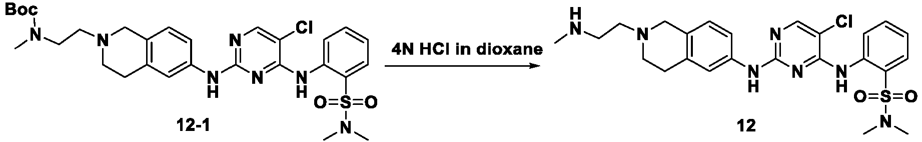

Compound 12-1 (350 mg, 0.6 mmol, 1.0 eq) was added to a solution of HCl in dioxane (4N, 6 mL), and the mixture reacted at room temperature for 12 hours. After the reaction was completed, the reaction solution was filtered to obtain a crude product. The crude product was purified by preparative liquid chromatography (acetonitrile/water, 0.1% formic acid as an additive, 20 mL/min) and lyophilized to obtain a formate of the target compound 12 (90 mg) as a yellow solid with a yield of 29.1%.

¹H NMR (400 MHz, DMSO-*d*₆): *δ* 9.45 (s, 1H), 9.28 (brs, 1H), 8.53 (brs, 1H), 8.31 (s, 1H), 8.28 (s, 1H), 7.83 (dd, *J* = 8.0, 1.2 Hz, 1H), 7.72-7.68 (m, 1H), 7.39-7.31 (m, 3H), 6.93 (d, *J =* 8.4 Hz, 1H), 3.54 (s, 2H), 2.99-2.96 (m, 2H), 2.78-2.66 (m, 6H), 2.64 (s, 6H), 2.48 (s, 3H).

LCMS: 516.2 ([M-HCOOH+H]⁺).

### Example 13

### Synthesis of 2-((5-chloro-2-((2-(2-(dimethylamino)ethyl)-1,2,3,4-tetrahydroisoquinolin-6-yl)amino)pyrimidin-4-yl)amino)-N,N dimethylbenzenesulfonamide formate (Compound 13)

### Step 1: Synthesis of 2-(dimethylamino)acetaldehyde (13-1)

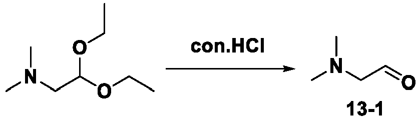

2,2-diethoxy-*N*,*N*-dimethylethane-1-amine (1.0 g, 6.2 mol) was dissolved in concentrated HCl (5 mL), and the mixture was heated to 40°C and reacted for 3 hours. The completion of the reaction was monitored by TLC. The reaction system was concentrated under reduced pressure to obtain the crude product 13-1 (200 mg), which was directly used in the next step of reaction.

### Step 2: Synthesis of 2-((5-chloro-2-((2-(2-(dimethylamino)ethyl)-1,2,3,4-tetrahydroisoquinolin-6-yl)amino)pyrimidin-4-yl)amino)-N,N dimethylbenzenesulfonamide formate (Compound 13)

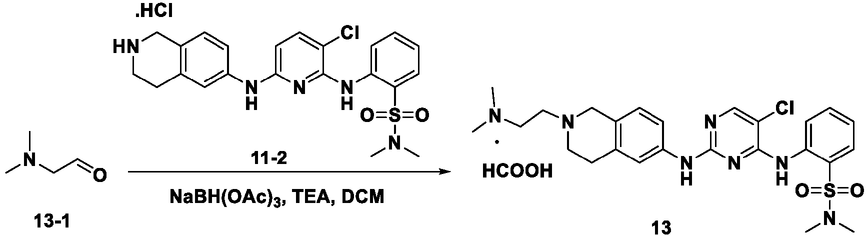

Compound 13-1 (45.6 mg, 0.52 mmol, 1.5 eq) was dissolved in 5 mL of DCM, compound 11-2 (160 mg, 0.35 mmol, 1.0 eq) and TEA(105 mg, 1.05 mmol, 3.0 eq) were added thereto, and the mixture reacted at room temperature for 10 minutes. Then, NaBH(OAc)₃ (133 mg, 0.7 mmol, 2.0 eq) was added, and after the addition was completed, the mixture reacted at room temperature for 30 minutes. The completion of the reaction was monitored by LC-MS. The reaction system was added with 10 mL of water, and then extracted with EtOAc for three times (15 mL each time). The organic phases were combined, washed twice with saturated brine (10 mL each time), and then dried over anhydrous sodium sulfate, filtered, and concentrated under reduced pressure to obtain a crude product. The crude product was separated and purified by preparative liquid chromatography (acetonitrile/water, 0.1% formic acid as an additive, 20 mL/min) to obtain the target compound 13 (26 mg, formate) as a yellow solid with a yield of 15.1%.

¹H NMR (400 MHz, DMSO-*d*₆): *δ* 9.43 (s, 1H), 9.27 (s, 1H), 8.57-8.49 (m, 1H), 8.48-8.12 (m, 3H), 7.82 (d, *J =* 7.6 Hz, 1H), 7.70 (t, *J =* 7.6 Hz, 1H), 7.39-7.35 (m, 2H), 7.30 (d, *J* = 8.4 Hz, 1H), 6.91 (d, *J* = 8.4 Hz, 1H), 3.53 (s, 2H), 2.75-2.67 (m, 4H), 2.64 (s, 6H), 2.58-2.52 (m, 4H), 2.22 (s, 6H).

LCMS: 530.2 ([M-HCOOH+H]⁺).

### Example 14

### Synthesis of 3-((5-chloro-2-((4-((4-methylpiperazin-1-yl)methyl)phenyl)amino)pyrimidin-4-yl)amino)-N,N-dimethylpyridine-4-sulfonamide (Compound 14)

### Step 1: Synthesis of 4-(phenylmethylthio)-3-bromopyridine (14-1)

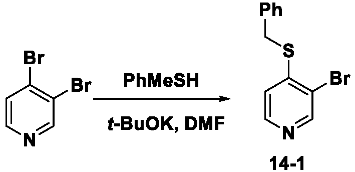

Compound 3,4-dibromopyridine (20.0 g, 84.4 mmol, 1.0 eq) and t-BuOK (15.0 g, 134 mmol, 1.6 eq) were dissolved in 200 mLDMF, and added with PhMeSH (15.0 g, 121 mmol, 1.4 eq) dropwise at 0°C. The mixture reacted at room temperature for 3 hours. The reaction solution was concentrated. The residue was diluted with 300 mL of water and extracted for three times with DCM. The organic phases were combined, washed with saturated brine, dried, filtered, and concentrated to obtain a crude product. The crude product was purified by silica gel column (PE: EtOAc = 5: 1) to obtain the target compound 14-1 (13.5 g, 48.1 mmol) as a white solid with a yield of 57.1%.

¹H NMR (300 MHz, CDCl₃):δ 8.47 (s, 1H), 8.26 (d, *J =* 5.3 Hz, 1H), 7.31 (dt, *J =* 10.1, 4.9 Hz, 5H), 7.00 (d, *J* = 5.3 Hz, 1H), 4.14 (s, 2H).

### Step 2: Synthesis of 3-bromopyridine-4-sulfonyl chloride (14-2)

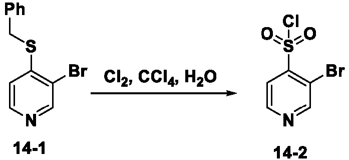

Compound 14-1 (6.00 g, 21.4 mmol) was dissolved in 72.0 mL of CCl₄ and 24.0 mL of H₂O. At 0°C, Cl₂ was added until the reaction system turned yellow. The mixture was added into water and extracted with DCM for three times. The organic phases were combined and washed with saturated brine to obtain the target compound 14-2, which was used directly in the next step.

### Step 3: Synthesis of 3-bromo-N,N-dimethylpyridine-4-sulfonamide (14-3)

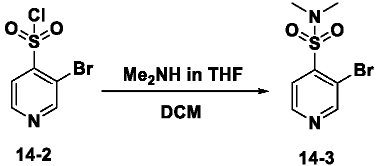

A solution of Me₂NH in THF was added to compound 14-2, and after the addition was completed, the mixture reacted at room temperature for 2 hours. The reaction solution was poured into water and extracted with DCM. The organic phases were combined, washed with saturated brine, and concentrated to obtain a crude product. The crude product was purified by silica gel column (PE: EtOAc = 3: 1) to obtain the target compound 14-3 (450 mg, 1.66 mmol) as a yellow solid with a yield of 3.13%.

¹H NMR (400 MHz, CDCl₃): δ 8.94 (s, 1H), 8.72 (d, *J =* 4.8 Hz, 1H), 7.88 (d, *J =* 5.2 Hz, 1H), 2.94 (s, 6H).

### Step 4: Synthesis of tert-butyl-(4-(N,N-dimethylaminosulfonyl)pyridin-3-yl)carbamate (14-4)

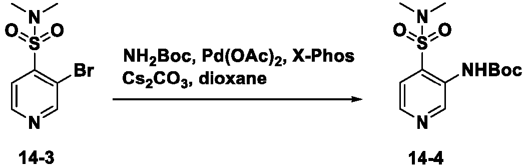

Compound 14-3 (450 mg, 1.69 mmol), NH₂Boc (270 mg, 2.45 mmol), Cs₂CO₃ (123 mg, 2.07 mmol), X-Phos (18.0 mg, 0.338 mmol), and Pd(OAc)₂ (10 mg, 0.169 mmol) were dissolved in 3 mL of dioxane. Under nitrogen protection, the temperature was raised to 130°C, and the mixture reacted for 1.5 hours. The reaction solution was filtered and concentrated to obtain a crude product. The crude product was purified by silica gel column to obtain the target compound 14-4 (300 mg, 0.997 mmol) as a yellow solid with a yield of 58.9%.

¹H NMR (400 MHz, CDCl₃): δ 9.60 (s, 1H), 8.49 (s, 1H), 8.45 (d, *J* = 6.8 Hz, 1H), 7.53 (d, *J =* 6.8 Hz, 1H), 2.94 (s, 6H), 1.53 (s, 9H).

LCMS: 302.1 ([M+H]⁺).

### Step 5: Synthesis of 3-amino-N,N-dimethylpyridine-4-sulfonamide (14-5)

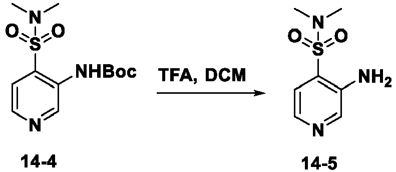

Compound 14-4 (0.3 g, 0.997 mmol) was dissolved in 2 mL of dry DCM, 2.00 mL of TFA was added thereto, and the mixture reacted at room temperature for 2 hours. The reaction solution was poured into water and adjusted to pH 8 with sodium bicarbonate, and extracted for three times with DCM. The organic phases were combined, washed with saturated brine, and concentrated to obtain the crude product 14-5 (210 mg) as a yellow solid.

¹H NMR (400 MHz, CDCl₃): δ 8.25 (s, 1H), 8.03 (d, *J =* 6.8 Hz, 1H), 7.33 (d, *J =* 6.8 Hz, 1H), 5.19(s, 2H),2.80 (s, 6H).

### Step 6: Synthesis of 3-((2,5-dichloropyrimidin-4-yl)amino)-N,N-dimethylpyridine-4-sulfonamide (14-6)

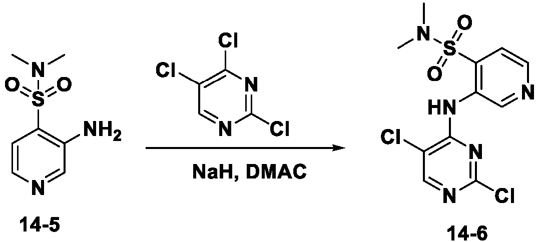

Compound 14-5 (210 mg, 1.04 mmol) was dissolved in 12.0 mL of DMAC, NaH (160 mg, 3.13 mmol) was added thereto at 25°C, and the mixture reacted at room temperature for 2 hours. Then, a solution of 2,4,5-trichloropyrimidine (160 mg, 0.03 mol) in DMAC (2 mL) was added thereto, and the mixture reacted at room temperature for 3 hours. The mixture was poured into water and methyl tert-butyl ether, and they were reacted at 25°C for 30 minutes. The reaction solution was filtered, and the residue was dried to obtain the target compound 14-6 (0.30 g) as a yellow solid with a yield of 83.2%.

¹H NMR (400 MHz, CDCl₃): δ 9.56 (s, 1H), 9.32 (s, 1H), 8.70 (d, *J =* 6.8 Hz, 1H),8.55(s, 1H), 7.79 (d, *J =* 6.8 Hz,1H) 2.71 (s, 6H).

### Step 7: Synthesis of 3-((5-chloro-2-((4-((4-methylpiperazin-1-yl)methyl)phenyl)amino)pyrimidin-4-yl)amino)-N,N-dimethylpyridine-4-sulfonamide (Compound 14)

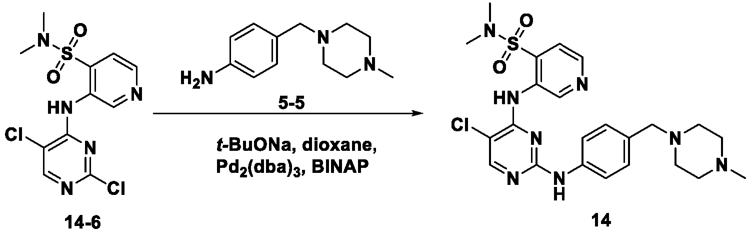

Compound 14-6 (140 mg, 0.403 mmol), compound 5-5 (112 mg, 0.630 mmol), and t-BuONa (49.0 mg, 0.504 mmol) were dissolved in 3.00 mL of dioxane. Under nitrogen protection, Pd₂(dba)₃ (0.0403 mmol) and BINAP (3.60 mg, 0.0806 mmol) were added thereto, and the mixture reacted in a microwave reactor at 130°C for 1 hour. The reaction solution was poured into water and then extracted with DCM. The organic phases were combined, washed with saturated brine, dried, and concentrated to obtain a crude product. The crude product was purified by preparative liquid chromatography to obtain the target compound 14 (24.5 mg) as a white solid.

¹H NMR (400 MHz, CDCl₃): δ 9.77 (s, 1H), 9.60(s, 1H), 9.17(s, 1H), 8.62(d, *J* = 5.2 Hz,1H), 8.30 (s, 1H), 8.62(d, *J =* 5.2 Hz,1H), 7.74 (d, *J =* 6.8 Hz,2H), 7.15(d, *J =* 5.2 Hz,1H),3.34(d, *J* = 6.8 Hz,2H) ,2.60(m,6H), 2.50(m,8H),2.32 (s, 3H).

LCMS: 517.1 ([M+H]⁺).

### Example 15

### Synthesis of 2-((5-chloro-2-((4-((3-methyl-3,8-diazabicyclo[3.2.1]octan-8-yl)methyl)phenyl)amino)pyrimidin-4-yl)amino)-N-methylbenzenesulfonamide (Compound 15)

### Step 1: Synthesis of 2-nitro-N-methylbenzenesulfonamide (15-2)

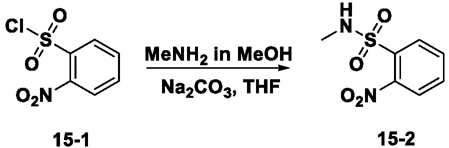

2-nitrobenzenesulfonyl chloride (15-1, 2.22 g, 10.0 mmol, 1.0 eq) was dissolved in 50 mL of THF, and sodium carbonate (2.1 g, 20.0 mmol, 2.0 eq) and 30% concentration of methylamine alcohol solution (1.5 g, 14.5 mmol, 1.45 eq) were added. The mixture was stirred and reacted at 60°C for about 2 hours. The completion of the reaction was monitored by TLC. The reaction solution was filtered, and the filtrate was spun-dried to obtain a yellow solid. The target compound 15-2 (2.16 g) was obtained as a yellow solid with a yield of 100%.

### Step 2: Synthesis of 2-amino-N-methylbenzenesulfonamide (15-3)

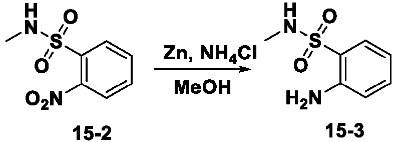

Compound 15-2 (2.16 g, 10.0 mmol) was dissolved in 50.0 mL of MeOH, and 3.9 g of Zn powder and 3.2 g of ammonium chloride were added thereto. The mixture reacted at 60°C for 8 hours. The filtrate was spun-dried, with appropriate amount of ethyl acetate (EA) and water being added, extracted and separated, washed with saturated brine, dried over sodium sulfate, and concentrated to obtain the target compound 15-3 (1.62 g) with a yield of 87.1%.

### Step 3: Synthesis of 2-((2,5-dichloropyrimidin-4-yl)amino)-N-methylbenzene sulfonamide (15-4)

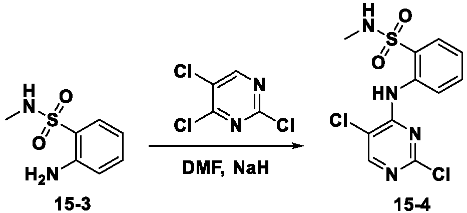

Compound 15-3 (1.62 g, 8.71 mol, 1.0 eq) was dissolved in 20 mL of anhydrous DMF, and NaH (1.5 g, 62.5 mmol, 7.2 eq) was added thereto at 25°C. After the addition was completed, the mixture reacted at room temperature for 4 hours and then cooled to 0°C in an ice-water bath. 0.75 g of NaH was added to a solution of 2,4,5-trichloropyrimidine (3.7 g, 20.3 mmol, 2.3 eq) in DMF (10 mL), and the mixture was added to the above reaction solution after appropriate stirring. The mixture reacted at 0°C for 0.5 hours, and the reaction solution was poured into 60 mL of ice water. The pH was adjusted to in the range of 4 to 6 with 3M HCl aqueous solution, and the mixture was extracted twice with EA, which was combined, washed with saturated brine, dried over sodium sulfate, and subjected to column chromatography (PE:EA=5: 1) to obtain the target compound 15-4 (0.66 g) as a white solid with a yield of 23.0%.

¹H NMR (300 MHz, DMSO-d6): δ 9.56 (s, 1H), 8.55 (s, 1H), 8.22 (dd, J = 8.3, 1.1 Hz, 1H), 7.86 (dd, J = 7.9, 1.6 Hz, 1H), 7.82 - 7.70 (m, 2H), 7.41 (td, J = 7.7, 1.2 Hz, 1H), 2.43 (d, J = 4.5 Hz, 3H).

### Step 4: Synthesis of 4-((5-chloro-4-((2-(N-methylaminosulfonyl)phenyl)amino)pyrimidin-2-yl)amino)methyl benzoate (15-5)

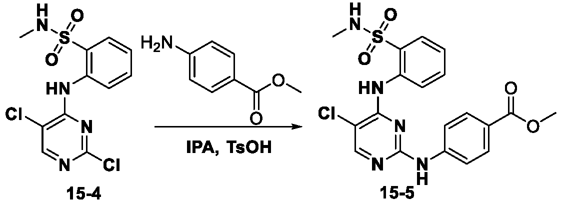

Compound 15-4 (0.66 g, 2 mmol, 1.0 eq) was dissolved in 240 mL of isopropanol, and methyl p-aminobenzoate (0.3 g, 2 mmol, 1.0 eq) and p-toluene sulfonic acid (0.52 g, 3 mmol, 1.5 eq) were added. The mixture reacted at 80°C for 48 hours, and then cooled to room temperature to precipitate a white solid. The solid was filtered and dried to obtain 0.48 g of compound 15-5 with a yield of 53.6%.

¹H NMR (300 MHz, DMSO-d6) : δ 9.97 (s, 1H), 9.28 (s, 1H), 8.45 (d, J = 8.3 Hz, 1H), 8.37 (s, 1H), 7.89 - 7.68 (m, 7H), 7.39 (td, J = 7.7, 7.3, 1.2 Hz, 1H), 3.81 (s, 3H), 2.44 (s, 3H).

### Step 5: Synthesis of 2-((5-chloro-2-((4-(hydroxymethyl)phenyl)amino)pyrimidin-4-yl)amino)-N-methylbenzenesulfonamide (15-6)

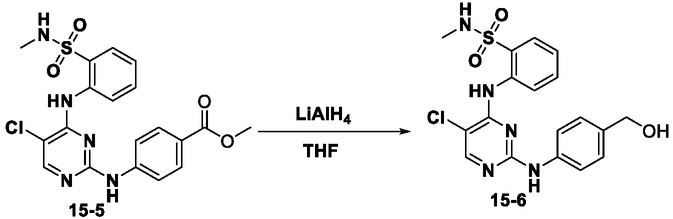

Compound 15-5 (0.48 g, 1.07 mmol, 1.0 eq) was dissolved in 60 mL of THF, and 0.8 mL of lithium aluminum hydride solution (2.5 mol/L dissolved in THF, 2 mmol, 2.1 eq) was added dropwise at 0°C. After the addition was completed, the mixture was returned to room temperature and reacted for 3 hours. The mixture was quenched with water, extracted with EA, and then subjected to column chromatography to obtain 0.15 g of compound 15-6 with a yield of 33.4%.

¹H NMR (300 MHz, DMSO-d6) : δ 9.51 (s, 1H), 9.29 (s, 1H), 8.54 (d, J = 8.4 Hz, 1H), 8.28 (s, 1H), 7.87 - 7.76 (m, 2H), 7.67 (ddd, J = 8.6, 7.4, 1.6 Hz, 1H), 7.58 (d, J = 8.5 Hz, 2H), 7.33 (ddd, J = 8.1, 7.4, 1.1 Hz, 1H), 7.19 (d, J = 8.5 Hz, 2H), 5.08 (t, J = 5.6 Hz, 1H), 4.43 (d, J = 5.1 Hz, 2H), 2.44 (d, J = 4.9 Hz, 3H).

### Step 6: Synthesis of 2-((5-chloro-2-((4-(chloromethyl)phenyl)amino)pyrimidin-4-yl)amino)-N-methylbenzenesulfonamide (15-7)

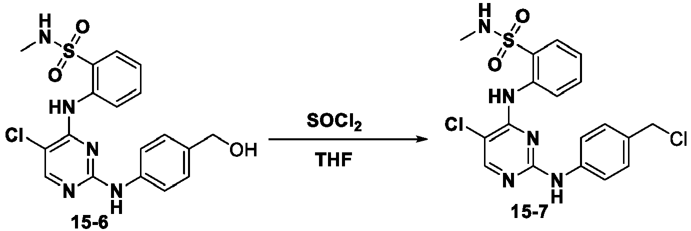

Compound 15-6 (0.15 g, 0.36 mmol, 1.0 eq) was dissolved in 6 mL of THF, 0.15 mL sulfoxide chloride (2 mmol, 5.5 eq) was added at room temperature, and the mixture reacted at 50 ° C for 4 hours. The mixture was spun-dried and added with an appropriate amount of EA. The mixture was extracted with saturated ammonium chloride solution, washed with saturated brine, dried over anhydrous sodium sulfate, and concentrated to obtain 0.16 g of compound 15-7 with a yield of 100%.

### Step 7: Synthesis of 2-((5-chloro-2-((4-((3-methyl-3,8-diazabicyclo[3.2.1]octan-8-yl)methyl)phenyl)amino)pyrimidin-4-yl)amino)-N-methylbenzenesulfonamide (15)

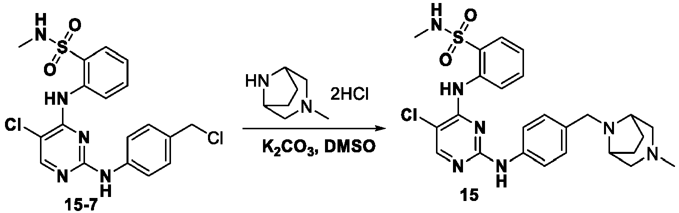

Compound 15-7 (0.16 g, 0.36 mmol, 1.0 eq) was dissolved in 2 mL of DMSO, and potassium carbonate (109 mg, 0.79 mmol, 2.2 eq) and 3-methyl-3,8-diazabicyclo[3.2.1]octane hydrochloride (73 mg, 0.36 mmol, 1.0 eq) were added. The mixture reacted at room temperature for 8 hours. An appropriate amount of saturated ammonium chloride solution was added, and the mixture was extracted with EA. The organic phases were combined, washed with saturated brine, dried over anhydrous sodium sulfate, and subjected to column chromatography (PE:EA=2:1, with 0.5/1000 triethylamine added) to obtain 80 mg of white solid compound 15 with a yield of 42.1%.

¹H NMR (400 MHz, DMSO-d6) : δ 9.47 (s, 1H), 9.31 (s, 1H), 8.56 (d, J = 8.4 Hz, 1H), 8.27 (s, 1H), 7.82 (dd, J = 8.0, 1.6 Hz, 2H), 7.67 - 7.59 (m, 1H), 7.59 (s, 2H), 7.31 (t, J = 7.6 Hz, 1H), 7.23 (d, J = 8.2 Hz, 2H), 3.40 (s, 3H), 3.03 (s, 2H), 2.50 - 2.47 (m, 1H), 2.45 (s, 3H), 2.16 (s, 1H), 2.13 (d, J = 4.2 Hz, 4H), 1.88 (dd, J = 8.5, 4.1 Hz, 2H), 1.71 (t, J = 6.2 Hz, 2H).

### Example 16

### Biological Test Evaluation

### 1. Cell viability assay for compounds of the present disclosure

### 1.1 Cell culture method

### 1.1.1 Culture of non-small cell lung cancer cell lines

Non-small cell lung cancer cell lines NCI-H1975, NCI-H1650, and NCI-H1792 were cultured in RPMI-1640 medium containing 10% fetal bovine serum and 1% penicillin-streptomycin. 1% sodium pyruvate was additionally added when culturing NCI-H1975 cells. All the cells were cultured in a constant temperature incubator containing 5% CO₂ at 37°C.

### 1.1.2 Culture of small cell lung cancer cell lines

Small cell lung cancer cell lines DMS114, NCI-H1688, NCI-H82, NCI-H146, and NCI-H446 cells were cultured in RPMI-1640 medium containing 10% fetal bovine serum and 1% penicillin-streptomycin. All the cells were cultured in a constant temperature incubator containing 5% CO2 at 37°C.

**1.1.3 Culture of primary cells from lung cancer pleural effusion**
(1) The pleural effusion was poured into a 15 mL or 50 mL of sterilized centrifuge tube for centrifugation. Centrifugation conditions: 800 to 1000 rpm, 5 min.
(2) The precipitate was resuspended and washed for 2 to 3 times with PBS.
(3) The cell precipitate was resuspended with complete culture medium (α-MEM medium containing 10% fetal bovine serum and 2% penicillin-streptomycin), and the flocculent matter in the pleural effusion was removed using a sterilized cell sieve.
(4) The red blood cells in the pleural effusion were removed with lymphocyte separation fluid, and the tumor cells and lymphocyte components were collected. The number of tumor cells was observed under a microscope, and the cells were inoculated.
(5) After 24 hours, the supernatant was discarded, and fresh culture medium was added for further culture.
(6) The cell growth status was observed, and it was determined based on the cell status whether the medium was required to be continuously changed to remove blood cells. When the cells grew to a density of 80% to 90%, the cells were preserved.

### 1.1.4 Culture of primary cells from lung cancer tissue

(1) The tissue blocks obtained from the tumor patients were transferred from the preservation solution to the pre-cooled HBSS buffer containing antibiotics, and the tissue blocks were washed for three times with the pre-cooled HBSS buffer.
(2) Tissue was sheared on ice with sterile surgical instruments until no obvious tissue blocks are visible to the naked eye, the tissue fluid was collected into a 15 ml centrifuge tube, and this process takes about 5 to 10 minutes.
(3) Digestive enzymes (collagenase I, collagenase IV and neutral protease being mixed in a ratio of 1:1:1, a concentration of stock solution was 10 mg/ml, diluted with PBS buffer or HBSS buffer, and the use concentration was 1 mg/ml) were added to the centrifuge tube, and after mixing well by pipetting, the centrifuge tube was placed in a 37°C constant temperature incubator for 1 to 2 hours. During this period, the centrifuge tube was shaken from time to time to mix and promote digestion until the tissue blocks are significantly reduced by naked eye observation, the digestion fluid is turbid, and a large number of floccules appear.
(4) High-glucose medium was added to the centrifuge tube to stop digestion. After mixing well, the cell precipitate was collected by centrifugation, and resuspended with MBM medium (Advanced DMEM-F12+20ng/ml bFGF+50ng/ml EGF+1%ITS-X+2%B27+10 µMY-27632+1%PS). The cell suspension was placed in a Matrigel-coated 24-well plate and cultured for 5 to 7 days. When the cell density in the well plate reaches 80-90%, the cells were subcultured into a 6 cm cell culture dish, and when they grew to a density of 80% to 90%, the cells were cryopreserved for preservation.

### 1.2 Detection of IC₅₀ of compounds for cell viability

### 1.2.1 Detection of IC₅₀ in non-small cell lung cancer cell lines

(1) The cells were cultured to the logarithmic growth phase. When the confluence reached about 90%, the cells were digested with 0.25% trypsin, and complete medium was added to collect the cells. After centrifugation at room temperature, the supernatant was discarded, and the cell precipitate was resuspended with fresh complete medium. After counting, the cells were placed at room temperature for later use.
(2) The cells were inoculated into a 96-well plate at a density of 4,000 cells/well with a volume of 100 µl/well. The wells at the outermost circle of the plate were not inoculated with cells, and instead, added with sterile water to reduce evaporation of the culture medium.
(3) After overnight culture of the cells, the medium was discarded, and a fresh medium containing different concentrations of compounds was added at 200 µl/well. The dilution method of the compound was as follows: first the compound with the highest concentration of 10 µM was prepared, and then the compound was diluted for 3 times in sequence. The final compound concentrations were set as follows: 10 µM, 3.33 µM, 1.11 µM, 0.37 µM, 0.12 µM, 0.041 µM, 0.014 µM, 0.0046 µM, 0.0015 µM, and 0 µM. The 0 µM group (negative control) was added with the compound solvent DMSO in the same proportion as that of the 10 µM group. The concentration of DMSO did not exceed 0.2%. At the same time, a blank control group was set up, in which only an equal volume of culture medium was added without adding cells. Six replicate wells were set up for each group. The cells were placed in the incubator for further culture.
(4) After 72 hours, 20 µl of MTT solution (5 mg/ml) sterilized by filtration through a 0.22 µm filter membrane was added to each well, and the cells were cultured in an incubator at 37°C for another 4 hours.
(5) The medium was carefully aspirated off, 100 µl of DMSO was added to dissolve the precipitate formed by the reaction with MTT, shaking and mixing to mix the blue-violet crystals well, and the light absorbance value at 492 nm was read with a microplate reader.
(6) IC₅₀ of the compounds for different cells was calculated based on the light absorption value of each group. Specifically, the absorbance value under treatment with different concentrations of compounds was first subtracted from the absorbance value of the blank well, and then the absorbance value of the 0 µM group was taken as 100% to calculate the cell survival rate after treatment with different concentrations of compounds. That is, survival rate = ((OD _{experimental well}-OD _{blank control})/(OD _{negative control}-OD _{blank control})) × 100%. **IC₅₀** values were calculated based on the survival rate and concentration of the compound.

### 1.2.2 Detection of IC₅₀ in primary cells from lung cancer pleural effusion

(1) Primary cells from lung cancer pleural effusion within 5 generations were cultured to the logarithmic growth phase. When the confluence reached about 90%, the cells were digested with 0.25% trypsin, and complete culture medium was added to collect the cells. After centrifugation at room temperature, the supernatant was discarded, and the cell precipitate was resuspended with fresh complete culture medium. After counting, the cells were placed at room temperature for later use. The cells were inoculated into a 384-well plate at a density of 2000 cells/well with a volume of 40 µl/well. The wells at the outermost circle of the plate were not inoculated with cells, and instead, added with sterile water to reduce evaporation of the culture medium.
(2) After overnight culture of the cells, the medium was discarded, and a fresh medium containing different concentrations of compounds was added at 200 µl/well. The dilution method of the compound was as follows: first the compound with the highest concentration of 10 µM was prepared, and then the compound was diluted for 3 times in sequence. The final compound concentrations were set as follows: 10 µM, 3.33 µM, 1.11 µM, 0.37 µM, 0.12 µM, 0.041 µM, 0.014 µM, 0.0046 µM, 0.0015 µM, and 0 µM. The 0 µM group (negative control) was added with the compound solvent DMSO in the same proportion as that of the 10 µM group. The concentration of DMSO did not exceed 0.2%. At the same time, a blank control group was set up, in which only an equal volume of culture medium was added without adding cells. Five replicate wells were set up for each group. The cells were placed in the incubator for further culture.
(3) After 72 hours, 8 µl of CCK-8 solution, which was double-diluted with 10 mM PBS, was added to each well, and the cells were shaken and placed in an incubator at 37°C for further culture for 2 hours.
(4) The light absorbance value at 450 nm was read using a microplate reader, and the **IC₅₀** was calculated according to the absorbance value. The calculation method was the same as above.

### 2. Detection of IC₅₀ of compounds for drug-resistant NSCLC cell lines

### 2.1 Detection of cell viability IC₅₀ of 10 compounds for Gefitinib-resistant NCI-H1975 cells

### (1) Detection of drug resistance in NCI-H1975 cells

NCI-H1975 cells were reported containing two mutation types, EGFR^{T790M} and EGFR^{L858R}, which were strains resistant to the first- and second-generation EGFR inhibitors, while sensitive to the third-generation inhibitor, i.e., Osimertinib. The first-generation EGFR inhibitor, Gefitinib, and the third-generation inhibitor, Osimertinib, were selected to treat NCI-H1975 cells, and IC₅₀ values thereof were determined. The results are shown in Table 1.

### (2) Detection of cell viability IC₅₀ of 10 compounds for NCI-H1975 cells

Cell viability IC₅₀ values of 10 compounds for NCI-H1975 cells were detected. The results are shown in Table 1.

### 2.2 Detection of cell viability IC₅₀ of Gefitinib-resistant and Osimertinib-resistant NCI-H1650 cells by 10 compounds

### (1) Detection of drug resistance in NCI-H1650 cells

NCI-H1650 cells were reported to have a mutation on the oncogene PTEN, and showed resistance to the first-, second-, and third-generation EGFR inhibitors. The first-generation EGFR inhibitor, Gefitinib, and the third-generation inhibitor, Osimertinib, were selected to treat NCI-H1650 cells, and IC₅₀ values thereof were determined. The results are shown in Table 1.

### (2) Detection of cell viability IC₅₀ of 10 compounds for NCI-H1650 cells

Cell viability IC₅₀ values of 10 compounds for NCI-H1650 cells were detected. The results are shown in Table 1.

**[Table 1] Detection results of cell viability IC₅₀ of the compounds of the present disclosure for NCI-H1975 and NCI-H1650 cells**

| Compound | IC₅₀ (nM) | |
|---|---|---|
| | NCI-H1975 | NCI-H1650 |
| Gefitinib | >10000 | 76692 |
| Osimertinib | 34.07 | 2749 |
| 2 | 86.37 | 466.3 |
| 3 | 28.33 | 37.76 |
| 4 | 87.67 | 163.4 |
| 6 | 47.73 | 250.0 |
| 8 | 55.17 | 83.63 |
| 9 | 25.28 | 170.0 |
| 10 | 21.48 | 135.7 |
| 11 | 29.15 | 32.55 |
| 12 | 18.56 | 75.19 |
| 13 | 19.77 | 67.31 |

The results in the above table indicate the following facts. In NCI-H1975 cells, the IC₅₀ of Gefitinib was greater than 10 µM, and the IC₅₀ of Osimertinib was 34.0nM, demonstrating that NCI-H1975 is a Gefitinib-resistant and Osimertinib-sensitive strain. In NCI-H1650 cells, the IC₅₀ of Gefitinib was greater than 10 µM, and the IC₅₀ of Osimertinib was 2749 nM, demonstrating that NCI-H1650 is a cell line resistant to both Gefitinib and Osimertinib. The pyrimidine-2,4-diamine derivatives represented by Formula 1 according to the present disclosure all had IC₅₀ less than 100 nM for the NCI-H1975 and NCI-H1650 cells resistant to EGFR-targeted medicaments, thereby having a good effect of inhibiting the growth of tumor cells.

### 2.3 Detection of cell viability IC₅₀ of compound 8 for NSCLC cells resistant to KRAS targeted drug AMG510

### (1) Detection of sensitivity of NCI-H1792 cells to KRAS G12C targeted medicaments

AMG510 is a novel small molecule drug targeting KRAS G12C. NCI-H1792 cells carry KRAS G12C mutation, but NCI-H1792 cells are resistant to AMG510. IC₅₀ of AMG510 in NCI-H1792 cells was detected by MTT method. The results are shown in Table 2.

### (2) Detection of cell viability IC₅₀ of compound 8 for NCI-H1792 cells

Cell viability IC₅₀ of compound 8 for NCI-H1792 cells was detected. The results are shown in Table 2.

**[Table 2] Detection results of cell viability IC₅₀ of compounds for NCI-H1792 cells**

| Compound | IC₅₀ (nM) |
|---|---|
| AMG510 | >10000 |
| 8 | 40.61 |

The detection results indicate that the NCI-H1792 cell line was resistant to AMG510, but sensitive to compound 8, and that the IC₅₀ of compound 8 was only 40.61 nM.

### 3. Cell viability IC₅₀ of compound 8 and compound 15 for small cell lung cancer cell lines

Small cell lung cancer is a highly heterogeneous malignant tumor, and its clinical treatment is mainly based on traditional chemotherapy (mainly platinum medicaments and etoposide). In order to study the anti-tumor activity of the compounds of the present disclosure in small cell lung cancer, the CCK-8 method was used to investigate the cell viability IC₅₀ of compound 8 for five small cell lung cancer cell lines. The results are shown in Table 3:

**[Table 3] Detection results of cell viability IC₅₀ of compounds 8 and 15 for small cell lung cancer cell lines**

| Drug in SCLC cells (IC₅₀ nM) | 8 | 15 | TP-0903 | Cisplatin | Etoposide |
|---|---|---|---|---|---|
| DMS114 | 6.03 | 8.53 | 95.08 | 2147 | 917.2 |
| H146 | 9.92 | 16.16 | 1536 | 3889 | 434.5 |
| H82 | 5.15 | ND | 35.03 | 1763 | 545.4 |
| H1688 | 5.01 | 9.16 | 534.0 | 14110 | 739.1 |
| H446 | 7.58 | 6.54 | 276.1 | 559 | 1062 |

| | | | | | |
|---|---|---|---|---|---|
| ND means not detected. | | | | | |

The detection results indicate that compounds 8 and 15 exhibited high in vitro antitumor activity in small cell lung cancer cell lines, with IC₅₀ values as low as tens of nanomoles, while the IC₅₀ values of chemotherapy medicaments Cisplatin and etoposide were significantly higher than that of compound 8. At the same time, TP-0903, which has a similar structure to compounds 8 and 15, has an IC₅₀ of tens to hundreds of nanomoles for the cell viability of five small cell lung cancer cell lines, which is much higher than compounds 8 and 15.

The structure of TP-0903was:

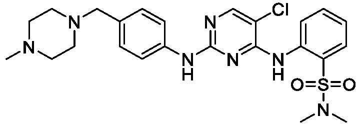

### 4. Detection of IC₅₀ of compound 8 for primary cells from lung cancer pleural effusion and tissues

### 4.1 Cell viability IC₅₀ of compound 8 for primary cells from pleural effusion and tissues of patients with small cell lung cancer

(1) Sample information: PE039, PE052, and PE062 were all primary cell samples from pleural effusion of patients with small cell lung cancer, and LT015 was a primary cell sample from bronchoscopic biopsy of a patient with small cell lung cancer.
(2) CCK-8 detection was used to detect the IC₅₀ values of different medicaments (compounds). The results are shown in Table 4.

**[Table 4] Cell viability IC₅₀ of compound 8 for primary cells from small cell lung cancer pleural effusion and tissue**

| Compound | IC₅₀ (nM) | | | |
|---|---|---|---|---|
| | PE039 | PE052 | PE062 | LT015 |
| 8 | 51.53 | 59.9 | 2.7 | 6.11 |
| TP-0903 | 133.9 | 210.3 | 5.1 | 94.28 |
| Cisplatin | 11,130 | 885 | 3,211 | 5,464 |
| Etoposide | ND | ND | 770.1 | 48,181 |
| Anlotinib | ND | 9,436 | 1,503 | 2,066 |
| Crizotinib | ND | ND | 900.2 | ND |
| Osimertinib | 3,991 | 568.2 | 2,738 | ND |
| AMG510 | ND | >10,000 | >10,000 | ND |

| | | | | |
|---|---|---|---|---|
| ND means not detected. | | | | |

The detection results indicate that compound 8 exhibited high anti-tumor activity in primary cells from pleural effusion of patients with small cell lung cancer, with an IC₅₀ ranging from 2.7 nM to 59.9 nM, while the IC₅₀ of chemotherapy medicaments, i.e., Cisplatin and etoposide, were both above hundreds of nanomoles. TP-0903, which is structurally analogous to compound 8, also had a higher IC₅₀ than compound 8 in primary cells PE039, PE052, and LT015.

### 4.2 Cell viability IC₅₀ of compound 8 for primary cells from non-small cell lung cancer pleural effusion resistant to EGFR-TKI

(1) PE043 sample information: the pathological result was lung adenocarcinoma; the patient's genetic detection results showed EGFR 19exon mutation and EGFR^{T790M} mutation; the clinical treatment process showed resistance to EGFR-TKI Gefitinib and Osimertinib, and resistance to chemotherapy medicaments Carboplatin and Pemetrexed.
(2) CCK-8 was used to detect the IC₅₀ values of different medicaments (compounds). The results were shown in Table 5.

**[Table 5] Detection results of cell viability IC₅₀ of compound 8 for primary cells from lung cancer pleural effusion resistant to EGFR-TKI**

| Compound | IC₅₀ (nM) |
|---|---|
| | PE043 |
| Gefitinib | 11,854 |
| Osimertinib | 3,373 |
| Cisplatin | 30,970 |
| Gemcitabine | >834,000 |
| 8 | 34.64 |

The detection results showed that the pleural effusion primary cells PE043 from patients with lung adenocarcinoma were resistant to Gefitinib, Osimertinib, Cisplatin, and gemcitabine, but sensitive to compound 8, and the IC₅₀ of compound 8 was only 34.64 nM.

It can be concluded from the above results that the compounds according to the present disclosure have good inhibitory effects on advanced drug-resistant NSCLC cell lines and small cell lung cancer cells. It can be concluded that the compounds according to the present disclosure may be used to treat advanced tumors, particularly advanced lung cancer, and further particularly small cell lung cancer and non-small cell lung cancer resistant to targeted drug treatment.

### 5. Detection of in vivo antitumor effect of compound 8 in mice bearing small cell lung cancer cell line H446 cells

### 5.1 Establishment of H446 cell CDX model

H446 cells in the logarithmic growth phase were suspended in PBS solution, and then inoculated subcutaneously in the right forelimb of nude mice. The cell density was about 2×10⁸ cells/mL, and each mouse was inoculated with 0.1 mL of the cell suspension, for a total of 80 mice. When the subcutaneous tumor grew to range from 50 mm³ to 100 mm³, 40 tumor-bearing mice were randomly divided into groups and numbered, and drug administration began. The number of each group was as follows: vehicle blank control group, 8 mice; experimental group of compound 8, 12 mice; chemotherapy drug control group, 8 mice; and TP-0903 control group, 12 mice.

### 5.2 Methods of drug administration and dosing regimen

(1) Vehicle blank control group: 5% DMSO + 20% PEG 300 + 75% saline (normal saline);
(2) Experimental group of compound 8 (15 mg/kg): 15 mg of compound 8 + 5% DMSO (0.375 mL) + 20% PEG 300 (1.5 mL) + 75% saline (5.625 mL) were prepared into a total volume of 7.5 mL of solution. The dosage for mice was 7.5 mL/kg, and the drug was administered by intraperitoneal injection once a day for 13 consecutive days, followed by 7 days of drug withdrawal.
(3) Control group of chemotherapy drug: Cisplatin 2.5 mg/kg, Etoposide 4 mg/kg, prepared as above, administered by intraperitoneal injection, 7 days as a cycle, Cisplatin 2.5 mg/kg on day 1 + Etoposide 4 mg/kg on days 1, 2, and 3, no drug on the remaining 4 days. The cycle repeated twice, and no medication for the final 6 days.
(4) Control group of TP-0903 (15 mg/kg): 15 mg/kg TP-0903, prepared as above, the dosing volume for mice was 7.5 mL/kg, injected intraperitoneally, once a day, for 13 consecutive days, and then stopped for 7 days.

### 5.3 Experimental process:

Once the mice were administrated with drug, the mice's condition was observed daily, their weight changes and tumor growth were monitored, and the curves of weight change and tumor volume growth were drawn. All tumor-bearing mice were killed after 20 days.

### 5.4 Deaths

(1) Blank control group: All 8 animals survived without death;
(2) Experimental group of compound 8: 11 out of 12 animals survived, and 1 died on the 14-th day;
(3) Control group of chemotherapy drug: 7 of 8 animals survived, and 1 died on the 15-th day;
(4) Control group of TP-0903: 11 of 12 animals survived, and 1 died on the 7-th day;

### 5.5 Changes in tumor size

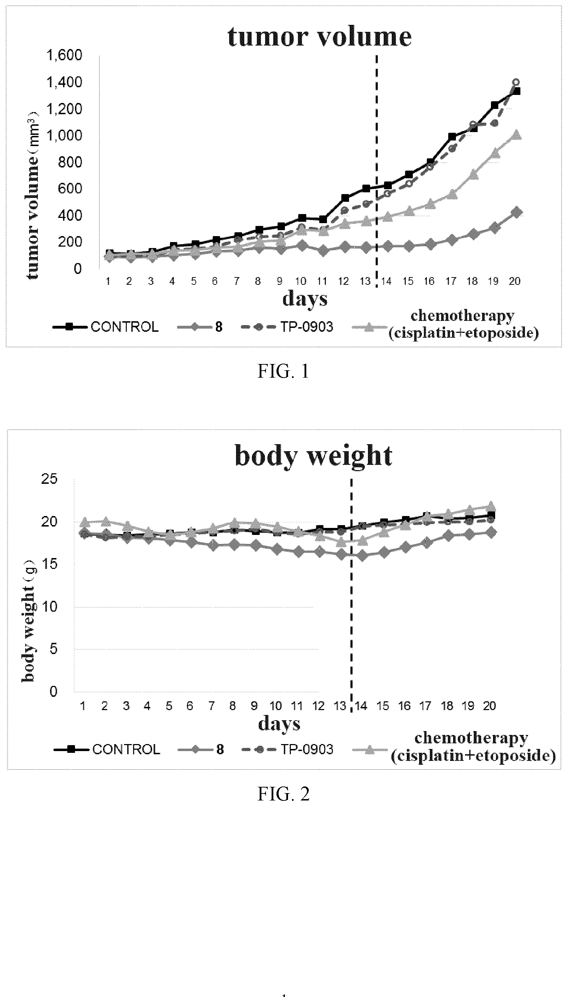

As shown in FIG. 1, compared with the blank control (CONTROL) group, the control group of TP-0903 had no significant inhibitory effect on tumor volume growth, while the experimental group of compound 8 and the control group of chemotherapy drug both significantly inhibited the growth of tumor volume, and the inhibitory effect of compound 8 on tumor volume growth was significantly stronger than that of chemotherapy medicaments.

### 5.6 Body weight changes of nude mice

As shown in FIG. 2, there was no significant decrease in body weight of nude mice in the blank control (CONTROL) group and the control group of TP-0903. There was a slight decrease in body weight of nude mice in the experimental group of compound 8 and the control group of chemotherapy drug during the drug administration period. However, the body weight recovered rapidly after drug withdrawal, indicating that the toxicity of compound 8 was not significant and was clinically acceptable.

### 6. Detection of cell viability IC₅₀ of compound 8 for various cancer cell lines

### 6.1 Experimental methods

The cancer cell lines were cultured to the logarithmic growth phase according to conventional culture methods, and the cells were inoculated into a 384-well plate at a density of 2,000 cells/well in a volume of 40 µl/well. The concentrations of added compounds were: 5 µM, 1.67 µM, 0.56 µM, 0.19 µM, 0.062 µM, 0.021 µM, 0.0069 µM, 0.0023 µM, 0.00076 µM, and 0 µM. After 72 hours, cell viability was detected using the CellTiter-Glo^{™} Detection Kit.

### 6.2 Experimental Results

The detection results of IC₅₀ were shown in Table 6.

**[Table 6] Detection results of cell viability IC₅₀ of compound 8 for other cancer cell lines**

| IC₅₀ value of compound 8 (nM) | |
|---|---|
| HCT116 (colon cancer) | 9.0 |
| SW620 (colon cancer) | 101.3 |
| HT29 (colon cancer) | 190.2 |
| Cal-62 (thyroid cancer) | 21.9 |
| ASH-3 (thyroid cancer) | 44.0 |
| MDA-MB-231 (breast cancer) | 49.9 |
| MCF-7 (breast cancer) | 52.8 |
| Hep3B (liver cancer) | 184.7 |
| Hela (cervical cancer) | 122.8 |
| RT-4 (bladder cancer) | 54.0 |
| OV-90 (ovarian cancer) | 103.4 |
| LN-18 (glioma) | 7.8 |
| MiaPaca-2 (pancreatic cancer) | 13.2 |
| KYSE-70 (esophageal cancer) | 122.1 |
| A375 (melanoma) | 23.3 |
| PC-3 (prostate cancer) | >3,000 |
| AGS (gastric cancer) | 36.2 |

The detection results showed that the IC₅₀ of compound 8 for thyroid cancer, breast cancer, bladder cancer, glioma, pancreatic cancer, melanoma, and gastric cancer was less than 100 nM, indicating that compound 8 has good anti-tumor potential for these tumors. In addition, the IC₅₀ for colon cancer, liver cancer, cervical cancer, ovarian cancer, and esophageal cancer was less than 200nM, indicating that compound 8 may also have some anti-tumor potential for these tumors.

In the specification, the description of the reference terms such as "one embodiment", "some embodiments", "example", "specific example", or "some example" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. A compound represented by Formula 1, or a racemate, tautomer, enantiomer, diastereomer, isotope-substituted compound, prodrug or pharmaceutically acceptable salt thereof, 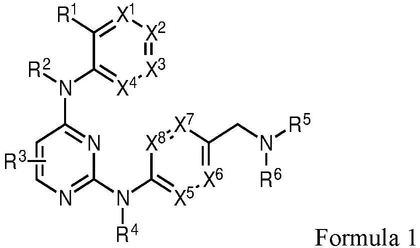 wherein:
X¹, X², X³, X⁴, X⁵, X⁶, X⁷, or X⁸ is each independently selected from N or CR⁷;
R¹ is selected from 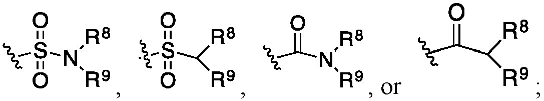
R² is selected from hydrogen, alkyl, or substituted alkyl;
R³ is selected from hydrogen, halogen, C₁ to C₆ alkyl, amino, (C₁ to C₆ alkyl)ₘ amino, (C₁ to C₆ alkyl)ₘ aminoalkyl, (C₁ to C₆ alkyl)ₘ amido, or (C₁ to C₆ alkyl)ₘ aminoacyl;
R⁴ is selected from hydrogen, alkyl, or substituted alkyl;
R⁵ and R⁶, together with N to which R⁵ and R⁶ are commonly bonded, form a 5-membered or 6-membered heterocyclic ring or a bridged ring; or R⁵ and R⁶, together with form a condensed ring; 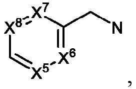
the 5-membered or 6-membered heterocyclic ring is selected from 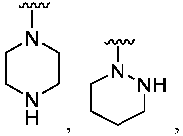 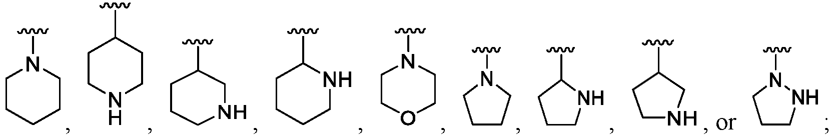
the bridged ring is selected from 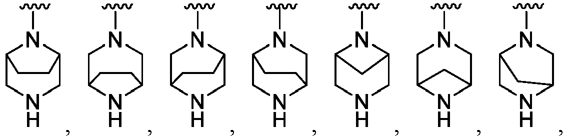 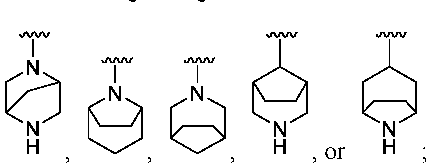
the condensed ring is selected from 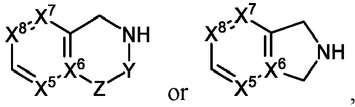 wherein Z or Y is each independently selected from a bond, (CH₂)ₙ, or NH;
the 5-membered or 6-membered heterocyclic ring, the bridged ring, or the condensed ring is each independently optionally substituted with one or more R¹⁰;
R⁸ and R⁹ are each independently selected from hydrogen, C₁ to C₆ alkyl, or C₃ to C₆ cycloalkyl;
R⁷ or R¹⁰ is each independently selected from hydrogen, C₁ to C₆ alkyl, halogenated C₁ to C₆ alkyl, hydroxyl C₁ to C₆ alkyl, C₁ to C₆ alkoxy, halogenated C₁ to C₆ alkoxy, halogen, cyano, hydroxyl, carboxyl, nitro, (C₁ to C₆ alkyl)ₘ aminocarbonyl, C₁ to C₆ alkylcarbonyl, (C₁ to C₆ alkyl)ₘ amino, (C₁ to C₆ alkyl)ₘ aminoalkyl, C₁ to C₆ alkoxyalkyl, (C₁ to C₆ alkyl)ₘ aminocarbonyl amino, sulfonic acid, C₁ to C₆ alkylsulfonyl, (C₁ to C₆ alkyl)ₘ aminosulfonyl, C₁ to C₆ alkylsulfonyl amino, or C₃ to C₆ cycloalkyl;
m is independently selected from 0, 1, or 2; and
n is 1 or 2.

2. The compound represented by Formula 1, or the racemate, tautomer, enantiomer, diastereomer, isotope-substituted compound, prodrug or pharmaceutically acceptable salt thereof according to claim 1, wherein:
R¹ is 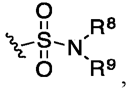 and preferably, R⁸ and R⁹ are each independently selected from H or methyl;
preferably, R² is hydrogen; and
preferably, R⁴ is hydrogen.

3. The compound represented by Formula 1, or the racemate, tautomer, enantiomer, diastereomer, isotope-substituted compound, prodrug or pharmaceutically acceptable salt thereof according to any one of claims 1 to 2, wherein R³ is halogen, and preferably, R³ is chlorine.

4. The compound represented by Formula 1, or the racemate, tautomer, enantiomer, diastereomer, isotope-substituted compound, prodrug or pharmaceutically acceptable salt thereof according to any one of claims 1 to 3, wherein:
the 5-membered or 6-membered heterocyclic ring is 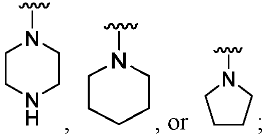
the bridged ring is 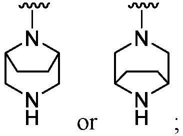 and
R¹⁰ is selected from C₁ to C₄ alkyl or (C₁ to C₆ alkyl)ₘ amino, m in R¹⁰ being 0, 1, or 2, and preferably, R¹⁰ is methyl or *N*,*N*-dimethylamino.

5. The compound represented by Formula 1, or the racemate, tautomer, enantiomer, diastereomer, isotope-substituted compound, prodrug or pharmaceutically acceptable salt thereof according to any one of claims 1 to 4, wherein:
X¹, X², X³, X⁴, X⁵, X⁶, X⁷, or X⁸ is CH; or
only one of X¹, X², X³, and X⁴ is N and/or only one of X⁵, X⁶, X⁷, and X⁸ is N.

6. The compound represented by Formula 1, or the racemate, tautomer, enantiomer, diastereomer, isotope-substituted compound, prodrug or pharmaceutically acceptable salt thereof according to any one of claims 1 to 5, wherein the compound represented by Formula 1, or the racemate, tautomer, enantiomer, diastereomer, isotope-substituted compound, prodrug or pharmaceutically acceptable salt thereof is a compound represented by Formula 2, 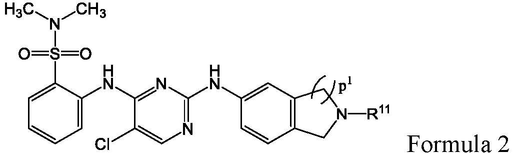 wherein:
R¹¹ is selected from C₁ to C₆ alkyl, hydroxyl C₁ to C₆ alkyl, C₁ to C₆ alkylcarbonyl, (C₁ to C₆ alkyl)ₘ aminoalkyl, or C₁ to C₆ alkoxyalkyl; and preferably, R¹¹ is selected from *N,N-*dimethylaminoethyl, methoxyethyl, and N-methylaminoethyl;
m is selected from 0, 1, or 2; and
p¹ is selected from 0, 1, 2, or 3.

7. The compound represented by Formula 1, or the racemate, tautomer, enantiomer, diastereomer, isotope-substituted compound, prodrug or pharmaceutically acceptable salt thereof according to any one of claims 1 to 5, wherein the compound represented by Formula 1, or the racemate, tautomer, enantiomer, diastereomer, isotope-substituted compound, prodrug or pharmaceutically acceptable salt thereof is a compound represented by Formula 3, 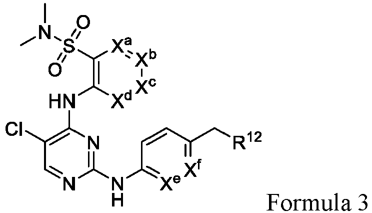 wherein:
X^{a}, X^{b}, X^{c}, X^{d}, X^{e}, or X^{f} is each independently selected from N or CH; or only one of X^{a}, X^{b}, X^{c}, and X^{d} is N and/or only one of X^{e} and X^{f} is N; and
R¹² is selected from 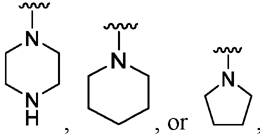 wherein R¹² is arbitrarily substituted with one or more substitutes of methyl, dimethylamine, or trimethylamine.

8. The compound represented by Formula 1, or the racemate, tautomer, enantiomer, diastereomer, isotope-substituted compound, prodrug or pharmaceutically acceptable salt thereof according to any one of claims 1 to 5, wherein the compound represented by Formula 1, or the racemate, tautomer, enantiomer, diastereomer, isotope-substituted compound, prodrug or pharmaceutically acceptable salt thereof is a compound represented by Formula 4, 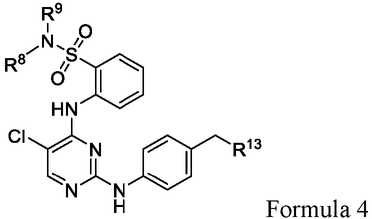 wherein:
R⁸ and R⁹ are each independently selected from hydrogen, C₁ to C₆ alkyl, or C₃ to C₆ cycloalkyl; and
R¹³ is selected from 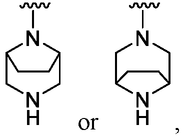 wherein R¹³ is arbitrarily substituted with one or more substitutes of methyl.

9. The compound represented by formula 1, or the racemate, tautomer, enantiomer, diastereomer, isotope-substituted compound, prodrug or pharmaceutically acceptable salt thereof according to any one of claims 1 to 8, wherein the compound represented by formula 1, or the racemate, tautomer, enantiomer, diastereomer, isotope-substituted compound, prodrug or pharmaceutically acceptable salt thereof is selected from the following compounds: 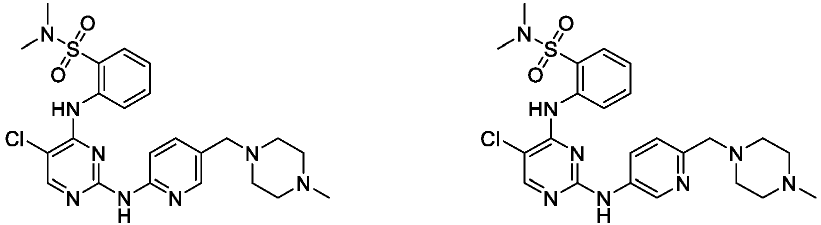 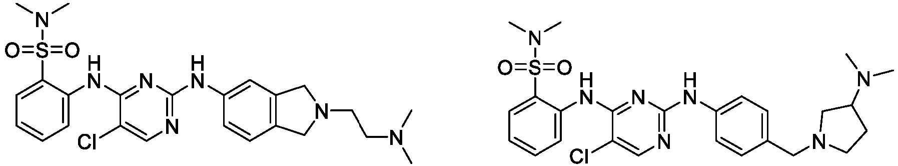 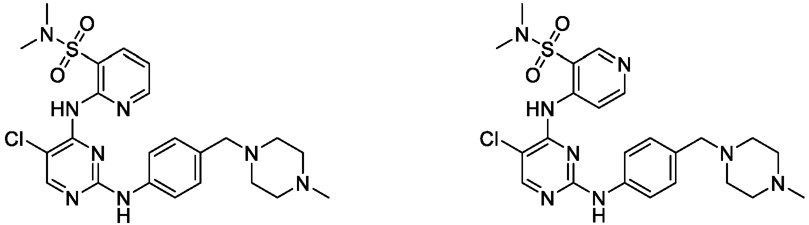 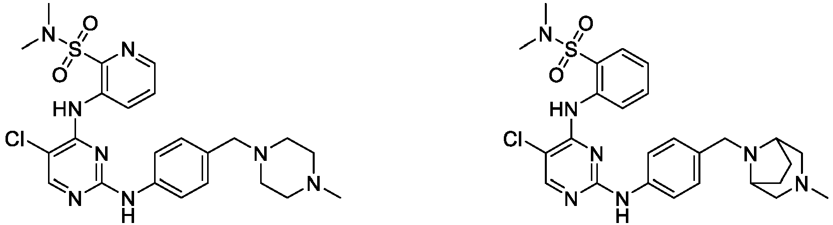 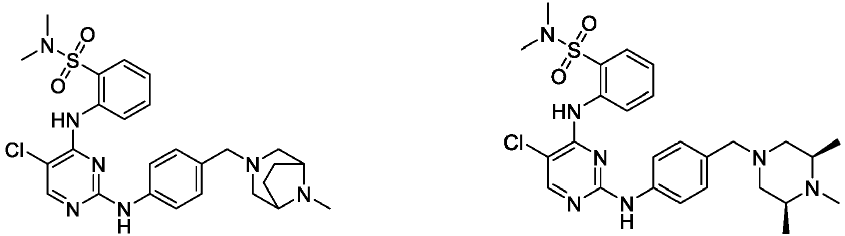 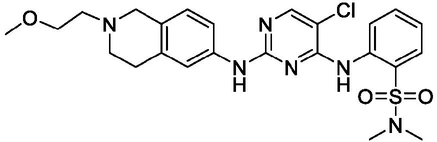 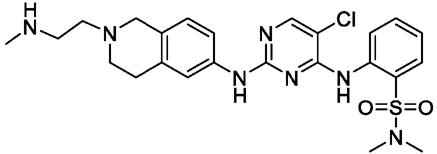 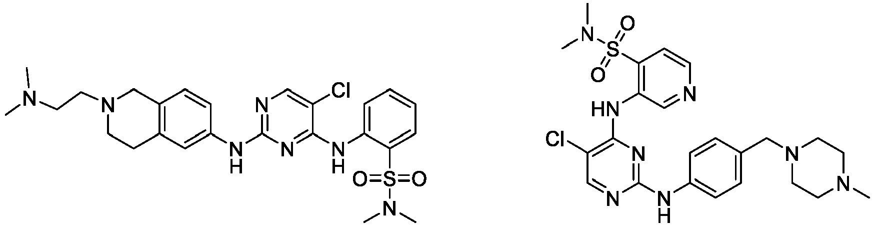 or

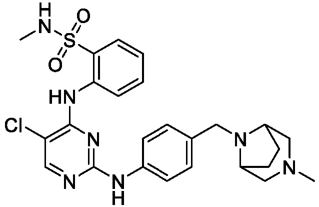

10. A method for preparing the compound represented by Formula 1 according to any one of claims 1 to 9, the method comprising:
1) performing a condensation reaction on an intermediate 1 and an intermediate 2 to generate an intermediate 3; and
2-1) performing a condensation reaction on the intermediate 3 and an intermediate 4 to generate the compound represented by Formula 1; or 3-1) performing a condensation reaction on the intermediate 3 and an intermediate 5 to generate an intermediate 6, and 3-2) performing a condensation reaction on the intermediate 6 and an intermediate 7 to generate the compound represented by Formula 1, according to the following reaction equation: 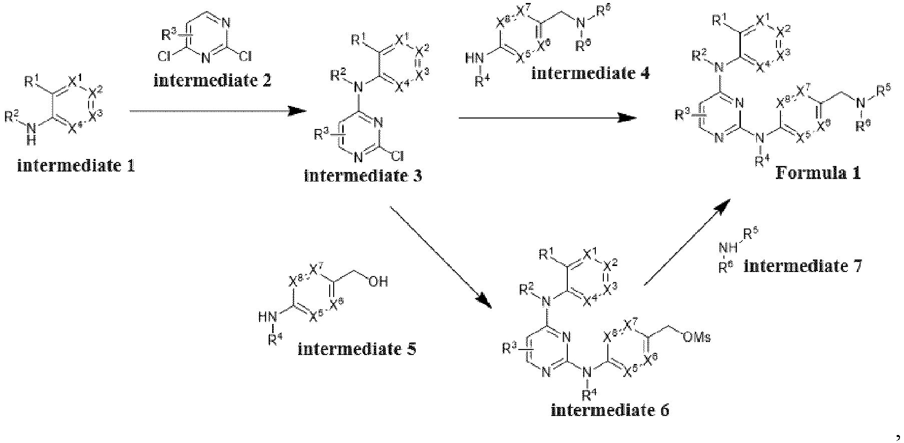 wherein X¹ to X⁸, R¹ to R⁶, m, and n are as defined in any one of claims 1 to 8.

11. A pharmaceutical composition, comprising:
a therapeutically effective dose of the compound represented by Formula 1, or the racemate, tautomer, enantiomer, diastereomer, isotope-substituted compound, prodrug or pharmaceutically acceptable salt thereof according to any one of claims 1 to 9; and
a pharmaceutically acceptable carrier or excipient.

12. Use of the compound represented by Formula 1, or the racemate, tautomer, enantiomer, diastereomer, isotope-substituted compound, prodrug or pharmaceutically acceptable salt thereof according to any one of claims 1 to 9, or the pharmaceutical composition according to claim 11 in the preparation of a medicament for treating a drug-resistant tumor.

13. Use of the compound represented by Formula 1, or the racemate, tautomer, enantiomer, diastereomer, isotope-substituted compound, prodrug or pharmaceutically acceptable salt thereof according to any one of claims 1 to 9, or the pharmaceutical composition according to claim 11 for the treatment of a drug-resistant tumor.

14. The use according to claim 12 or 13, wherein:
the drug-resistant tumor is an advanced solid tumor;
preferably, the advanced solid tumor is advanced lung cancer, advanced thyroid cancer, advanced bladder cancer, advanced glioma, advanced pancreatic cancer, advanced melanoma, advanced gastric cancer, advanced colorectal cancer, advanced liver cancer, advanced cervical cancer, advanced ovarian cancer, or advanced esophageal cancer; and
preferably, the advanced solid tumor is small cell lung cancer, or non-small cell lung cancer resistant to targeted drug therapy.

15. A method for treating a drug-resistant tumor, comprising:
administering to a patient a pharmaceutically acceptable dose of the compound represented by formula 1, or the racemate, tautomer, enantiomer, diastereomer, isotope-substituted compound, prodrug or pharmaceutically acceptable salt thereof according to any one of claims 1 to 9, or the pharmaceutical composition according to claim 11, wherein:
preferably, the drug-resistant tumor is an advanced solid tumor;
preferably, the advanced solid tumor is advanced lung cancer, advanced thyroid cancer, advanced bladder cancer, advanced glioma, advanced pancreatic cancer, advanced melanoma, advanced gastric cancer, advanced colorectal cancer, advanced liver cancer, advanced cervical cancer, advanced ovarian cancer, or advanced esophageal cancer; and
preferably, the advanced solid tumor is small cell lung cancer or non-small cell lung cancer resistant to targeted drug therapy.
